(19) Europäisches Patentamt
European Patent Office
Office européen des brevets

(11) **EP 4 674 407 A1**

(12) **EUROPEAN PATENT APPLICATION**
published in accordance with Art. 153(4) EPC

(43) Date of publication:
07.01.2026 Bulletin 2026/02

(51) International Patent Classification (IPC):
*A61K 9/107* (2006.01)  *A61K 47/00* (2006.01)
*A61K 8/64* (2006.01)  *A61K 38/48* (2006.01)

(21) Application number: 24763193.0

(22) Date of filing: 28.02.2024

(52) Cooperative Patent Classification (CPC):
A61K 8/34; A61K 8/36; A61K 8/49; A61K 8/64;
A61K 9/00; A61K 9/107; A61K 31/045;
A61K 36/064; A61K 36/238; A61K 36/484;
A61K 38/48; A61K 47/00; A61K 47/10;
A61K 47/12; A61K 47/22;          (Cont.)

(86) International application number:
PCT/CN2024/078986

(87) International publication number:
WO 2024/179513 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.02.2023 CN 202310178312

(71) Applicant: Kangma-Healthcode (Shanghai)
Biotech Co., Ltd
Pudong New Area
Shanghai 201321 (CN)

(72) Inventors:
• GUO, Min
  Shanghai (CN)
• JIANG, Zhuyan
  Shanghai (CN)

• LIU, Zhang
  Shanghai (CN)
• XIAO, Song
  Shanghai (CN)
• FENG, Yan
  Shanghai (CN)
• YU, Xue
  Shanghai (CN)

(74) Representative: Dai, Simin
Reyda IP
A073
157, Quai du Président Roosevelt
92130 Issy-les-Moulineaux (FR)

Remarks:
The complete document including Reference
Table(s) and the Sequence Listing(s) can be
downloaded from the EPO website

(54) **TRANSDERMAL COMPOSITION AND USE THEREOF**

(57) Disclosed are a transdermal composition capable of allowing an active substance to effectively transdermally exert a predetermined effect, and use thereof. The transdermal composition contains the active substance capable of exerting the predetermined effect. The transdermal composition is in the form of an emulsion. Based on the total weight of the transdermal composition in percentage by mass, the transdermal composition comprises 15%-50%, 30%-45% or 35%-40% of oil phase, 35%-65% or 40%-50% of emulsifier, and 10%-35% or 10%-25% of aqueous phase.

**FIG. 1**

(Cont. next page)

(52) Cooperative Patent Classification (CPC): (Cont.)
    **A61K 47/26; A61K 47/36; A61K 47/42;
    A61P 17/00; A61P 17/18; A61P 25/02;
    A61Q 19/00; A61Q 19/02; A61Q 19/08**

**Description**

TECHNICAL FIELD

The present disclosure belongs to the field of biotechnology, and particularly relates to a transdermal composition and use thereof.

BACKGROUND

Oral delivery is the most commonly used administration mode because of its convenience, safety and ease of patient acceptance. However, However, it faces challenges such as poor drug solubility and stability, degradation in the gastrointestinal tract, and hepatic first-pass effects, leading to low bioavailability.

Injection delivery can cause the fluctuation in blood drug concentration in a body. Due to short medicine half-life, pain at an injection site and other side effects will be caused after frequent injection delivery, with poor patient compliance. Currently, a majority of macromolecules are administered via injection, making long-term treatment difficult to maintain.

Transdermal drug delivery systems (TDDs for short) or transdermal therapeutic system (TTS for short) is a method in which drugs are absorbed via a skin, which is a new administration route in which drugs are absorbed via the skin into blood circulation of a human body so as to reach an effective blood concentration and achieve disease treatment or prevention.

The transdermal drug delivery system is the third largest drug delivery system following oral delivery and injection, which can avoid the first-pass effect of the liver and the destruction of the drugs caused by gastrointestinal enzymes, and has the advantages of convenient administration, low toxic and side effects and high safety.

In addition to treating common dermatoses, the transdermal drug delivery has also begun to be used to treat systemic diseases such as hypertension, diabetes and cancer, and has been applied to a medical beauty industry. Its application in the beauty industry brings many conveniences to patients and beauty lovers.

Currently, there have already been drugs with the transdermal drug delivery system available on the market, such as estradiol and testosterone, their delivery mainly targets the development of small molecules and lipophilic drugs.

As for macromolecular and hydrophilic components, it is difficult for them to penetrate the epidermis and dermis of the skin into the site of action to function due to the natural barrier effect of the skin.

SUMMARY

The present disclosure provides a transdermal composition capable of allowing an active substance, especially biomacromolecular and hydrophilic active substances, to effectively transdermally exert a predetermined effect, and use thereof.

To this end, the present disclosure provides the following technical solutions.

The present disclosure provides a transdermal composition, wherein the transdermal composition contains an active substance capable of exerting a predetermined effect; the transdermal composition is in the form of an emulsion; and based on the total weight of the transdermal composition in percentage by mass, in the transdermal composition:

the percentage of oil phase is: 15%-50%, 30%-45% or 35%-40%;
the percentage of emulsifier is: 35%-65% or 40%-50%;
the percentage of aqueous phase is: 10%-35% or 10%-25%.

After testing, the emulsion has a particle size distribution of 0.01 $\mu$m-1 $\mu$m.

The transdermal composition provided in the present disclosure also has the following characteristics: wherein, based on the total weight of the transdermal composition in percentage by mass, in the transdermal composition:

the percentage of oil phase is: 15%-50%, 30%-45% or 35%-40%;
the percentage of emulsifier is: 35%-65% or 40%-50%;
the percentage of aqueous phase is: 10%-35% or 10%-25%.

The transdermal composition provided in the present disclosure also has the following characteristics: wherein, a mass ratio of the oil phase to the aqueous phase to the emulsifier is: 2-3:2.4-4:1 or 2-3:2.4-3.7:1.

The transdermal composition provided in the present disclosure also has the following characteristics: based on the total weight of the transdermal composition in percentage by mass, a matrix component of the emulsion comprises: 1%-15% of oleic acid; 10%-30% of emulsifying stabilizer, 5%-15% of any one or more of propylene glycol and butylene glycol, 0.5%-4% of menthol, 1%-5% of laurocapram, 35%-65% of emulsifier, 10%-20% of water and other components.

The transdermal composition provided in the present disclosure also has the following characteristics: the emulsifier comprises sorbitan oleate and any one or more selected from polysorbate 80, polysorbate 60 and polysorbate 20, and in percentage by mass:

the sorbitan oleate accounts for 10%-15% of the total weight of the transdermal composition, and any one or more of polysorbate 80, polysorbate 60 and polysorbate 20 accounts for the total weight of the transdermal composition is 25%-50% or 30%-40%.

The transdermal composition provided in the present disclosure also has the following characteristics: based on the total weight of the transdermal composition in percentage by mass, the matrix component of the emulsion comprises: 1%-15% of oleic acid; 10%-30% of emulsion stabilizer, 5%-15% of any one or more of propylene glycol and butylene glycol, 0.5%-4% of menthol, 1%-5% of laurocapram, 35%-65% of emulsifier and 10%-20% of water.

The transdermal composition provided in the present disclosure also has the following characteristics: the transdermal composition contains a preservative,

preferably, based on the total weight of the transdermal composition in percentage by mass, the transdermal composition comprises 0.1%-1% of preservative. Furthermore, the preservative is selected from any one or more of phenoxyethanol, methylparaben, ethylparaben, propylparaben, benzyl alcohol and sorbic acid.

The transdermal composition provided in the present disclosure also has the following characteristics: the active substance contains a biomolecular substance, preferably, the transdermal composition can allow the biomolecule active substance of less than or equal to approximately 300 kDa or less than or equal to approximately 150 kDa to permeate.

The transdermal composition provided in the present disclosure also has the following characteristics: the predetermined effect is to achieve any one or more effects of treatment, cosmetic and skin care effects.

The transdermal composition provided in the present disclosure also has the following characteristics: the aforementioned biomolecular substance has a structure capable of achieving the following functions: an exocytosis fusion machinery capable of being transported to a target cell and cleaving the target cell.

Furthermore, the biomolecular substance is a clostridial neurotoxin.

Even furthermore, the clostridial neurotoxin is a type A, B, C, D, E, F or G botulinum toxin, and/or a light chain of the clostridial neurotoxin comprises an amino acid sequence having at least 35%, 50%, 60%, 70%, 75%, 80%, 85%, 90%, 99% or 100% identity to any one of SEQ ID NOs: 1-7; and/or a heavy chain of the clostridial neurotoxin comprises an amino acid sequence having at least 35%, 50%, 60%, 70%, 75%, 80%, 85%, 90%, 99% or 100% identity to any one of SEQ ID NOs: 8-14.

The transdermal composition provided in the present disclosure also has the following characteristics: the active substance comprises any one or more of the following: a yeast extract, a plant extract, and other substances that can exert any one or more effects of skin whitening, moisturizing, anti-aging, repairing and cell growth, and the components in the active substance are different from those in the foregoing matrix composition (note: the difference is made from the perspective view of functions mainly achieved for the main purpose of convenience, but is not limited: the matrix composition cannot have components that have the same effects as those of the active substance. That is to say, the matrix composition certainly contains components that have the same effects as those of the active substance. Only when it is mainly used as the component of the matrix, in order to distinguish the proportion description, the role as the component of the matrix is emphasized).

The transdermal composition provided in the present disclosure also has the following characteristics: the active substance comprises any one or more of the following: a yeast extract, sodium ascorbyl phosphate, *Hippophae rhamnoides* seed oil, optional vitamins, retinyl palmitate, an aloe extract, allantoin, *Glycyrrhiza glabra* extract, a ginseng extract, a *Rheum palmatum* extract, a *Centella asiatica* extract, a tea extract and a *Hamamelis virginiana* extract.

The transdermal composition provided by the present disclosure is characterized in that the transdermal composition has compositions as shown in Table 6 or Table 7.

The present disclosure further provides use of the aforementioned transdermal composition in achieving therapeutic, cosmetic and skin care effects.

The use provided by the present disclosure also has the following characteristics: when being used, transdermal use is performed in any one or more of applying, a transdermal instrument, patching and spraying.

The present disclosure further provides use of the aforementioned transdermal composition in the preparation of a medicament or product for achieving therapeutic, cosmetic and skin care effects.

The present disclosure further provides a preparation method of a transdermal composition as described above, comprising:

dissolving and mixing dissolved oil phase raw materials respectively, and then dissolving and mixing the obtained mixtures respectively to obtain a final oil phase mixture;
adding an aqueous phase raw material into the final oil phase mixture directly or after dissolving to be mixed.

The preparation method of the transdermal composition provided in the present disclosure also has the following characteristics:

wherein the composition of the transdermal composition comprises:
oleic acid, oleyl alcohol, propylene glycol, menthol, laurocapram, sorbitan oleate, any one or more of polysorbate 80, polysorbate 60 and polysorbate 20, water, and an effective amount of a biomolecule
the preparation method comprises:
obtaining a first oil phase group: uniformly mixing a quantitative polysorbate 80, polysorbate 60 and sorbitan oleate; then adding a quantitative oleyl alcohol and oleic acid to continue uniform mixing; and then adding a predetermined amount of laurocapram and phenoxyethanol to continue uniform mixing to obtain the first oil phase group;
obtaining a second oil phase group: mixing and fully dissolving a quantitative propylene glycol and menthol to obtain the second oil phase group;
obtaining a final oil phase mixture: uniformly mixing the first oil phase group with the second oil phase group to obtain the final oil phase mixture;
adding an effective amount of the biomolecule into the final oil phase mixture to be uniformly mixed, and supplementing water.

The preparation method of the transdermal composition provided in the present disclosure also has the following characteristics:

wherein the composition of the transdermal composition comprises:
oleic acid, oleyl alcohol, propylene glycol, menthol, laurocapram, sorbitan oleate, and any one or more of polysorbate 80, polysorbate 60 and polysorbate 20, water, and
a yeast extract, sodium ascorbyl phosphate, *Hippophae rhamnoidesseed* oil, optional vitamins, retinyl palmitate, an aloe extract, allantoin, a *Glycyrrhiza glabra* extract, a ginseng extract, a *Rheum palmatum* extract, a *Centella asiatica* extract, a tea extract, and a *Hamamelis virginiana* extract,
the preparation method comprises:
obtaining a first oil phase group: uniformly mixing a quantitative polysorbate 80, polysorbate 60 and sorbitan oleate; then adding a quantitative oleyl alcohol, oleic acid, *Hippophae rhamnoides* seed oil, tocopherol, and retinyl palmitate to continue uniform mixing; then adding a quantitative laurocapram and phenoxyethanol to continue uniform mixing to obtain the first oil phase group;
obtaining a second oil phase group: mixing and fully dissolving a quantitative propylene glycol and menthol to obtain the second oil phase group;
obtaining a final oil phase mixture: uniformly mixing the first oil phase group with the second oil phase group to obtain the final oil phase mixture;
separately adding a quantitative plant extract, yeast extract, and sodium ascorbyl phosphate, niacinamide, and allantoin separately dissolved in water to the final oil phase mixture sequentially, uniformly mixing, and then supplementing water.

**Functions and effects of the present disclosure**

The transdermal composition provided in the present disclosure is in the form of an emulsion, which can achieve effective transdermal delivery to exert a predetermined effect due to appropriate proportion ranges of the oil phase, the aqueous phase and the emulsifier in the emulsion, especially for macromolecular substances and hydrophilic substances, they can also achieve effective transdermal delivery to exert the predetermined effect.

BRIEF DESCRIPTION OF THE DRAWINGS

FIG. 1 is a particle transmission electron microscope picture of transdermal compositions prepared in Examples 1-6;
FIG. 2 is a graph showing the results of a drug efficacy verification experiment of transdermal compositions prepared in Examples 1-6 in mice;
FIG. 3 is a comparison chart of skin elasticity R2 values before and after a transdermal composition prepared in Example 8 is used (the larger the R2 measurement value, the better the skin elasticity);
FIG. 4 is a comparison chart of skin firmness F4 values before and after a transdermal composition prepared in Example 8 is used (the smaller the F4 value, the better the skin firmness);
FIG. 5 is a comparison chart of area ratios of under-eye wrinkles before and after a transdermal composition prepared in Example 8 is used (the smaller the under-eye measurement value, the smaller the area ratio of under-eye wrinkles);

**FIG. 6** is a comparison chart of clinical evaluation-facial fine line grades before and after a transdermal composition prepared in Example 8 is used (the smaller the measured value, the smaller the proportion of facial fine line area);
**FIG. 7** is a comparison chart of nasolabial fold grades, Chuan word pattern grades, forehead wrinkle grades and lip corner wrinkle grades before and after a transdermal composition prepared in Example 8 is used (the smaller the measured value, the smaller the proportion of fine lines); and

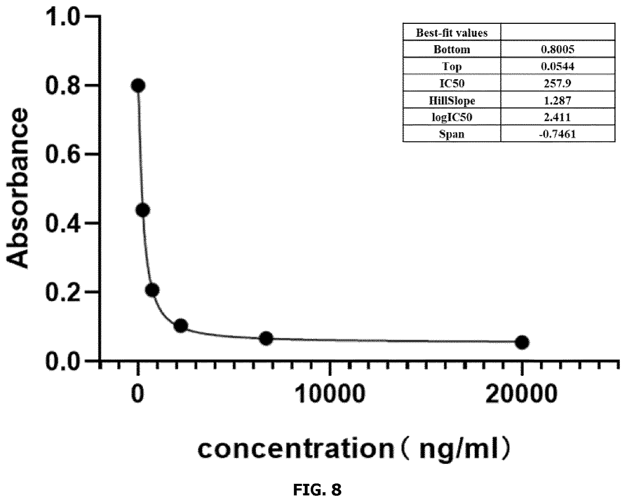

**FIG. 8** shows a transdermal experiment conducted on a transdermal composition.

DETAILED DESCRIPTION OF THE PREFERRED EMBODIMENT

Next, the specific embodiments of the present disclosure will be described with reference to accompanying drawings. The specific methods or materials used in embodiments undergo conventional replacement selection by Those skilled in the art according to the existing technology based on the technical ideas of the present disclosure, but are not limited to the specific description of the embodiments of the present disclosure.

Unless otherwise specified, the methods used in the embodiments are conventional methods; the materials and reagents used are all commercially available, unless otherwise specified.

An emulsion refers to a non-uniformly dispersed liquid formulation consisting of two immiscible liquid phases where one of the two phases is dispersed into the other phase in the form of small droplets. The phase forming the droplets is called a dispersed phase, an internal phase or a discontinuous phase, and the other phase is called a dispersion medium, an external phase or a continuous phase.

The emulsion consists of an oil phase (denoted by O), an emulsifier and an aqueous phase (denoted by W). Depending on the type, property and phase volume ratio ($\varphi$) of the emulsion, an oil-in-water (O/W) or water-in-oil (W/O) emulsion or a composite emulsion can be formed.

An aqueous phase refers to water or an aqueous solution. The aqueous solution means that a raw material that is easily dissolved into water is dissolved into water.

An oil phase means that a raw material that is difficultly dissolved into water is dissolved into an oily substance.

The transdermal composition provided in the present disclosure comprises an effective amount of active substance that can exert a predetermined effect. The transdermal composition is in the form of an emulsion: the active substance is incorporated into an emulsion matrix.

The predetermined effect is to achieve any one or more of treatment, beauty and skin care effects.

The emulsion matrix or excipient composition mentioned herein refers to an essential component that primarily forms an emulsion form relative to the active substance. Of course, the emulsion matrix may also comprise components that have the same effects as those of the active substance. While the emulsion matrix is specifically defined for illustrative purposes, the composition of the overall emulsion needs to comprehensively consider the emulsion matrix and other components, and conform to the proportions of the oil phase, emulsifier and aqueous phase of the present disclosure.

The transdermal composition of the present disclosure is defined as shown in Table 1 and Table 2:

| Table 1 | |
|---|---|
| In percentage by mass based on the total weight of the transdermal composition | |
| Oil phase | 15%-50%, or 30%-45%, or 35%-40%; |
| Emulsifier | 35%-65%, or 40%-50%; |
| Aqueous phase | 10%-35%, or 10%-25% |
| Preferably, the oil phase is 35%-40%, the emulsifier is 40%-50%, the aqueous phase is 10%-25%. | |

| Table 2 |
|---|
| By mass ratio |
| Oil phase : aqueous phase : emulsifier is: 2-3:2.4-4:1, or 2-3:2.4-3.7:1 |

In Tables 1 and 2, the entire transdermal composition consists of three types namely an oil phase, an aqueous phase and an emulsifier, in which "an effective amount of active substance capable of exerting a predetermined effect" is included, that is to say, the active substance may be in the oil phase or in the aqueous phase.

The emulsion of the present disclosure has a particle size distribution of 0.01 $\mu$m-1 $\mu$m or comprises particles with a particle size of 100-200 nm. Preferably, the emulsion of the present disclosure is water-in-oil.

Based on the total weight of the transdermal composition in percentage by mass, the emulsion matrix is shown in Table 3:

| Table 3 | | |
|---|---|---|
| Name of composition | Category | In percentage by mass based on the total weight of the transdermal composition |
| Oleic acid | Oil phase | 1%-15% |
| Emulsifying stabilizer (such as oleyl alcohol) | | 10%-30% |
| Propylene glycol or butylene glycol | | 5%-15% |
| Menthol | | 1.5%-9% 0.5%-4% |
| Laurocapram | | 1%-5% |
| Emulsifier (such as any one or more of Polysorbate 80, Polysorbate 60, Polysorbate 20 and sorbitan oleate) | Emulsifier | 35%-65% |
| Water | Aqueous phase | 10%-20% |

Furthermore, in percentage by mass:

The sorbitan oleate accounts for 10%-15% of the total weight of the transdermal composition, and any one or more of polysorbate 80, polysorbate 60 and polysorbate 20 accounts for 30%-40% of the total weight of the transdermal composition.

Furthermore, an example of the matrix components of the transdermal composition includes those as shown in Table 4:

| Table 4 | | |
|---|---|---|
| Name of composition | Category | In percentage by mass based on the total weight of the transdermal composition |
| Oleic acid | Oil phase | 1%-15% |
| Oleyl alcohol | | 10%-30% |
| Propylene glycol | | 5%-15% |
| Menthol | | 0.5%-4%, or 1%-2% |
| Laurocapram | | 1%-5 %, or 2%-4%, or 2% |
| Any one or more of Polysorbate 80, Polysorbate 60, and Polysorbate 20 | Emulsifier | 30%-40% |
| Sorbitan oleate | | 10%-15% |
| Water | Aqueous phase | 10%-20% |

In one example, the transdermal composition further comprises a preservative, preferably, based on the total weight of the transdermal composition in percentage by mass, the transdermal composition comprises 0.1%-1% of preservative. Furthermore, the preservative is selected from any one or more of phenoxyethanol, methylparaben, ethylparaben, propylparaben, benzyl alcohol and sorbic acid.

The active substance of the present disclosure comprises a biomolecular substance, preferably, the transdermal composition can allow the biomolecular active substance that is less than or equal to approximately 300 kDa or less than or equal to approximately150kDa to permeate. The "approximately" herein is intended to express in addition to a range less than or equal to, there is a relatively large range that is relatively close, for example, 305 KDa. Preferably, a range beyond 50 KDa is a proximity range, that is to say, less than or equal to 350 kDa, or less than or equal to 200 kDa.

A Da's full name is Dalton, which is a common unit representing a molecular weight, that is to say, an algebraic sum of atomic weights for all atoms in a molecule is calculated based on their number. Dalton (Da) is numerically equal to a relative molecular mass.

In biochemistry, molecular biology and proteomics, kDa (kilodalton) is often used to represent biological macromolecules such as proteins. 1 kDa represents a molecule with a relative molecular mass of 1000. A macromolecule refers to a biological substance with a relative molecular mass of 5000 or more, or even more than one million, such as a peptide, a protein, a nucleic acid, a polysaccharide and an antibody.

In one example, the biomolecular substance has a structure capable of achieving the following functions: an exocytosis fusion machinery capable of being transported to a target cell and cleave the target cell.

Specifically, a non-cytotoxic protease cleaves an intracellular transport protein called a SNARE protein so as to exert the predetermined effect by proteolysis (see Gerald K (2002)-Cell and Molecular Biology (4th edition) John Wiley & Sons, Inc.).

The acronym SNARE is derived from a term soluble NSF attachment receptor, in which NSF represents N-ethylmaleimide-sensitive factor.

The SNARE protein is necessary for an intracellular vesicle fusion body and is therefore essential for the secretion of a molecule from cells via vesicle transport. The function of the non-cytotoxic protease is zinc-dependent endopeptidase activity and exhibits high substrate specificity for the SNARE protein. The SNARE protein is associated with a membrane or cell membrane of a secretory vesicle and promotes the exocytosis of a molecule by mediating the fusion of the secretory vesicle with the cell membrane, thereby allowing the contents of the vesicle to be excreted out of the cells. The cleavage of the neuron SNARE prevents the release of neurotransmitters, thereby resulting in paralysis. Thus, once the non-cytotoxic protease is delivered to the desired target cells, it can inhibit cell secretion by the desired target cells.

There proteins at the ends of the synapse, synaptobrevin VAMP on the synaptic vesicle membrane as well as syntaxin and SNAP-25 on the presynaptic membrane are all referred to as SNARE proteins.

In one example, the cell is a neuron cell, and the protein of the exocytosis fusion machinery is the SNARE protein of the neuron cell. By cleaving the SNARE protein, for example, by preventing the release of the acetylcholine neurotransmitter, the physiological function of cholinergic nerve conduction is blocked.

Further, the biomolecular substance is a clostridial neurotoxin.

The clostridial toxins can be used for therapeutic and cosmetic purposes. For example, by specifically binds to a presynaptic receptor on peripheral cholinergic nerve endings and by cleaving the synaptic-associated protein SNAP-25 botulinum toxin interferes with the exocytosis of the presynaptic vesicles, inhibits the release of acetylcholine (ACh) from nerve endings and causes chemical denervation of muscles, thereby resulting in muscle relaxation and relief of spasms. The muscle chemical denervation effect of the botulinum toxin is a theoretical basis for adjustment of hypertonia, movement disorders and dynamic wrinkles. Furthermore, multiple injections of the botulinum toxin into target muscles can lead to disuse atrophy of muscles and then reduction in muscle volume, and therefore the botulinum toxin is also used for body shaping. Currently, the application of the botulinum toxin in the field of disease treatment includes the treatment of hemifacial spasm, essential blepharospasm, spastic torticollis, spastic cerebral palsy, post-stroke limb spasticity, tremor and other various dystonias diseases. The application of the botulinum toxin in the field of minimally invasive plastic surgery includes the removal of various dynamic wrinkles such as frown lines, forehead lines and crow's feet; the beautification of facial contours such as eyebrow height adjustment, mental muscle relaxation, mandibular margin lifting and neck cord injection; and the shaping of body contours such as masseter muscle reduction, gastrocnemius muscle reduction and trapezius muscle reduction.

The clostridial toxin herein refers to for example a natural clostridial toxin and has a similar structure and functions similar to those of the natural clostridial toxins.

Despite there are differences in amino acid sequences and immunogenicity of various types of toxins, they exhibit similar molecular structures. The clostridial neurotoxin is produced by toxigenic Clostridium species in a form of a non-toxic single-chain polypeptide of approximately 150 kD. The produced clostridial neurotoxin has activity after being cleaved by a bacterial protease or an in vitro protease into a two-chain form, i.e., a light chain (L chain, the amino terminus of the toxin, 50 kD) linked by a disulfide bond and a heavy chain (H chain, the carboxyl terminus of the toxin, 100 kD). The heavy chain is composed of two domains: Hn (at the amino terminus, 50 kD) and Hc (at the carboxyl terminus, 50 kD).

Furthermore, the clostridial neurotoxin is a type A, B, C, D, E, F or G botulinum toxin, and/or the light chain of the clostridial neurotoxin comprises an amino acid sequence having at least 35%, 50%, 60%, 70%, 75%, 80%, 85%, 90%, 99%, or 100% identity to any one of SEQ ID NOs: 1-7; and/or the heavy chain of the clostridial neurotoxin comprises an amino acid sequence having at least 35%, 50%, 60%, 70%, 75%, 80%, 85%, 90%, 99%, or 100% identity to any one of SEQ ID NOs: 8-14. The sequences are seen in Table 5.

| Table 5 Sequence Listing | |
|---|---|
| SEQ ID NO:1 | MPFVNKQFNYKDPVNGVDIAYIKIPNVGQMQPVKAFKIHNKIWVIPERDTFTNPEEGD LNPPPEAKQVPVSYYDSTYLSTDNEKDNYLKGVTKLFERIYSTDLGRMLLTSIVRGIPF WGGSTIDTELKVIDTNCINVIQPDGSYRSEELNLVIIGPSADIIQFECKSFGHEVLNLTR NGYGSTQYIRFSPDFTFGFEESLEVDTNPLLGAGKFATDPAVTLAHELIHAGHRLYGIAI NPNRVFKVNTNAYYEMSGLEVSFEELRTFGGHDAKFIDSLQENEFRLYYYNKFKDIAST LNKAKSIVGTTASLQYMKNVFKEKYLLSEDTSGKFSVDKLKFDKLYKMLTEIYTEDNFVK FFKVLNRKTYLNFDKAVFKINIVPKVNYTIYDGFNLRNTNLAANFNGQNTEINNMNFTK LKNFTGLFEFYKLLCVRGIITSQTQS |
| SEQ ID NO:2 | MPVTINNFNYNDPIDNNNIIMMEPPFARGTGRYYKAFKITDRIWIIPERYTFGYKPEDF NKSSGIFNRDVCEYYDPDYLNTNDKKNIFLQTMIKLFNRIKSKPLGEKLLEMIINGIPYL GDRRVPLEEFNTNIASVTVNKLISNPGEVERKKGIFANLIIFGPGPVLNENETIDIGIQNH FASREGFGGIMQMKFCPEYVSVFNNVQENKGASIFNRRGYFSDPALILMHELIHVLHGL YGIKVDDLPIVPNEKKFFMQSTDAIQAEELYTFGGQDPSIITPSTDKSIYDKVLQNFRGI VDRLNKVLVCISDPNININIYKNKFKDKYKFVEDSEGKYSIDVESFDKLYKSLMFGFTET NIAENYKIKTRASYFSDSLPPVKIKNLLDNEIYTIEEGFNISDKNMEKEYRGQNKAINKQ AYEEISKEHLAVYKIQMCKSVR |
| SEQ ID NO:3 | MPITINNFNYSDPVDNKNILYLDTHLNTLANEPEKAFRITGNIWVIPDRFSRNSNPNLN KPPRVTSPKSGYYDPNYLSTDSDKDTFLKEIIKLFKRINSREIGEELIYRLSTDIPFPGNN NTPINTFDFDVDFNSVDVKTRQGNNWVKTGSINPSVIITGPRENIIDPETSTFKLTNNT FAAQEGFGALSIISISPRFMLTYSNATNDVGEGRFSKSEFCMDPILILMHELNHAMHNLY GIAIPNDQTISSVTSNIFYSQYNVKLEYAEIYAFGGPTIDLIPKSARKYFEEKALDYYRSIA KRLNSITTANPSSFNKYIGEYKQKLIRKYRFVVESSGEVTVNRNKFVELYNELTQIFTEFN YAKIYNVQNRKIYLSNVYTPVTANILDDNVYDIQNGFNIPKSNLNVLFMGQNLSRNPAL RKVNPENMLYLFTKFCHKAIDGR |

(continued)

| Table 5 Sequence Listing | |
|---|---|
| SEQ ID NO:4 | MTWPVKDFNYSDPVNDNDILYLRIPQNKLITTPVKAFMITQNIWVIPERFSSDTNPSLS KPPRPTSKYQSYYDPSYLSTDEQKDTFLKGIIKLFKRINERDIGKKLINYLVVGSPFMGD SSTPEDTFDFTRHTTNIAVEKFENGSWKVTNIITPSVLIFGPLPNILDYTASLTLQGQQS NPSFEGFGTLSILKVAPEFLLTFSDVTSNQSSAVLGKSIFCMDPVIALMHELTHSLHQLY GINIPSDKRIRPQVSEGFFSQDGPNVQFEELYTFGGLDVEIIPQIERSQLREKALGHYKD IAKRLNNINKTIPSSWISNIDKYKKIFSEKYNFDKDNTGNFVVNIDKFNSLYSDLTNVMS EVVYSSQYNVKNRTHYFSRHYLPVFANILDDNIYTIRDGFNLTNKGFNIENSGQNIERN PALQKLSSESVVDLFTKVCLRLTKNSR |
| SEQ ID NO:5 | MPTINSFNYNDPVNNRTILYIKPGGCQQFYKSFNIMKNIWIIPERNVIGTIPQDFLPPTS LKNGDSSYYDPNYLQSDQEKDKFLKIVTKIFNRINDNLSGRILLEELSKANPYLGNDNT PDGDFIINDASAVPIQFSNGSQSILLPNVIIMGAEPDLFETNSSNISLRNNYMPSNHGFG SIAIVTFSPEYSFRFKDNSMNEFIQDPALTLMHELIHSLHGLYGAKGITTKYTITQKQNP LITNIRGTNIEEFLTFGGTDLNIITSAQSNDIYTNLLADYKKIASKLSKVQVSNPLLNPYK DVFEAKYGLDKDASGIYSVNINKFNDIFKKLYSFTEFDLATKFQVKCRQTYIGQYKYFKL SNLLNDSIYNISEGYNINNLKVNFRGQNANLNPRIITPITGRGLVKKIIRFCKNIVSVKGI R |
| SEQ ID NO:6 | MPVAINSFNYNDPVNDDTILYMQIPYEEKSKKYYKAFEIMRNVWIIPERNTIGTNPSDF DPPASLKNGSSAYYDPNYLTTDAEKDRYLKTTIKLFKRINSNPAGKVLLQEISYAKPYLG NDHTPIDEFSPVTRTTSVNIKLSTNVESSMLLNLLVLGAGPDIFESCCYPVRKLIDPDVV YDPSNYGFGSINIVTFSPEYEYTFNDISGGHNSSTESFIADPAISLAHELIHALHGLYGAR GVTYEETIEVKQAPLMIAEKPIRLEEFLTFGGQDLNIITSAMKEKIYNNLLANYEKIATRL SEVNSAPPEYDINEYKDYFQWKYGLDKNADGSYTVNENKFNEIYKKLYSFTESDLANKF KVKCRNTYFIKYEFLKVPNLLDDDIYTVSEGFNIGNLAVNNRGQSIKLNPKIIDSIPDKGL VEKIVKFCKSVIPRKGTK |

(continued)

| Table 5 Sequence Listing | |
|---|---|
| SEQ ID NO:7 | MPVNIKXFNYNDPINNDDIIMMEPFNDPGPGTYYKAFRIIDRIWIVPERFTYGFQPDQF NASTGVFSKDVYEYYDPTYLKTDAEKDKFLKTMIKLFNRINSKPSGQRLLDMIVDAIPYL GNASTPPDKFAANVANVSINKKIIQPGAEDQIKGLMTNLIIFGPGPVLSDNFTDSMIMN GHSPISEGFGARMMIRFCPSCLNVFNNVQENKDTSIFSRRAYFADPALTLMHELIHVLH GLYGIKISNLPITPNTKEFFMQHSDPVQAEELYTFGGHDPSVISPSTDMNIYNKALQNF QDIANRLNIVSSAQGSGIDISLYKQIYKNKYDFVEDPNGKYSVDKDKFDKLYKALMFGF TETNLAGEYGIKTRYSYFSEYLPPIKTEKLLDNTIYTQNEGFNIASKNLKTEFNGQNKAV NKEAYEEISLEHLVIYRIAMCKPVMYKNTGK |
| SEQ ID NO:8 | ALNDLCIKVNNWDLFFSPSEDNFTNDLNKGEEITSDTNIEAAEENISLDLIQQYYLTFNF DNEPENISIENLSSDIIGQLELMPNIERFPNGKKYELDKYTMFHYLRAQEFEHGKSRIAL TNSVNEALLNPSRVYTFFSSDYVKKVNKATEAAMFLGWVEQLVYDFTDETSEVSTTDKI ADITIIIPYIGPALNIGNMLYKDDFVGALIFSGAVILLEFIPEIAIPVLGTFALVSYIANKVLT VQTIDNALSKRNEKWDEVYKYIVTNWLAKVNTQIDLIRKKMKEALENQAEATKAIINY QYNQYTEEEKNNINFNIDDLSSKLNESINKAMININKFLNQCSVSYLMNSMIPYGVKRL EDFDASLKDALLKYIYDNRGTLIGQVDRLKDKVNNTLSTDIPFQLSKYVDNQRLLSTFT EYINNIINTSILNLRYESNHLIDLSRYASKINIGSKVNFDPIDKNQIQLFNLESSKIEVILK NAIVYNSMYENFSTSFWIRIPKYFNSISLNNEYTIINCMENNSGWKVSLNYGEIIWTLQ DTQEIKQRVVFKYSQMINISDYINRWIFVTITNNRLNNSKIYINGRLIDQKPISNLGNIH ASNNIMFKLDGCRDTHRYIWIKYFNLFDKELNEKEIKDLYDNQSNSGILKDFWGDYLQ YDKPYYMLNLYDPNKYVDVNNVGIRGYMYLKGPRGSVMTTNIYLNSSLYRGTKFIIKKY ASGNKDNIVRNNDRVYINVVVKNKEYRLATNASQAGVEKILSALEIPDVGNLSQVVVM KSKNDQGITNKCKMNLQDNNGNDIGFIGFHQFNNIAKLVASNWYNRQIERSSRTLGC SWEFIPVDDGWGERPL |

(continued)

| Table 5 Sequence Listing | |
|---|---|
| SEQ ID NO:9 | APGICIDVDNEDLFFIADKNSFSDDLSKNERIEYDTQSNYIENRSSINELILDTNLISKIE LPSENTESLTDFNVDVPVYEKQPAIKKIFTDENTIFQYLYSQTFPLDIRDISLTSSFDDAL LFSNKVYSFFSMDYIKTANKVVEAGLFAGWVKQIVDDFVIEANKSSTMDKIADISLIVPY IGLALNVGNETAKGNFENAFEIAGASILLEFIPELLIPVVGAFLLESYIDNKNKIIKTIDNA LTKRDEKWIDMYGLIVAQWLSTVNTQFYTIKEGMYKALNYQAQALEEIIKYKYNIYSEK EKSNINIDFNDINSKLNEGINQAIDNINDFINECSVSYLMKKMIPLAVEKLLDFDNTLKK NLLNYIDENKLYLIGSAEYEKSKVDKYLKTIIPFDLSMYTNNTILIEIFNKYNSEILNNIILN LRYRDNNLIDLSGYGANVEVYDGVKLNDKNQFKLTSSTNSEIRVTQNQNIIFNSMFLDF SVSFWIRIPKYKNDGIQNYIHNEYTIINCIKNNSGWKISIRGNRIIWTLTDINGKTKSVF FEYSIREDISDYINRWFFVTITNNSDNAKIYINGKLESNIDIKNIGEVIANGEIIFKLDGDI DRTQFIWMKYFSIFNTELSQSNIKEIYKIQSYSEYLKDFWGNPLMYNKEYYMFNAGNK NSYIKLKKDSSVGEILTRSKYNQNSNYINYRNLYIGEKFIIRRKSNSQSINDDIVRKEDYI YLDFFNSNREWRVYAYKDFKEEEKKLFLANIYDSNEFYKTIQIKEYDEQPTYSCQLLFKK DEESTDEIGLIGIHRFYESGIVLKDYKNYFCISKWYLKEVKRKPYNPNLGCNWQFIPKD EGWIE |

(continued)

| Table 5 Sequence Listing | |
|---|---|
| SEQ ID NO:10 | SLYNKTLDCRELLVKNTDLPFIGDISDVKTDIFLRKDINEETEVIYYPDNVSVDQVILSKN TSEHGQLDLLYPSIDSESEILPGENQVFYDNRTQNVDYLNSYYYLESQKLSDNVEDFTF TRSIEEALDNSAKVYTYFPTLANKVNAGVQGGLFLMWANDVVEDFTTNILRKDTLDKIS DVSAIIPYIGPALNISNSVRRGNFTEAFAVTGVTILLEAFPEFTIPALGAFVIYSKVQERNE IIKTIDNCLEQRIKRWKDSYEWMMGTWLSRIITQFNNISYQMYDSLNYQAGAIKAKID LEYKKYSGSDKENIKSQVENLKNSLDVKISEAMNNINKFIRECSVTYLFKNMLPKVIDEL NEFDRNTKAKLINLIDSHNIILVGEVDKLKAKVNNSFQNTIPFNIFSYTNNSLLKDIINEY FNNINDSKILSLQNRKNTLVDTSGYNAEVSEEGDVQLNPIFPFDFKLGSSGEDRGKVIV TQNENIVYNSMYESFSISFWIRINKWVSNLPGYTIIDSVKNNSGWSIGIISNFLVFTLKQ NEDSEQSINFSYDISNNAPGYNKWFFVTVTNNMMGNMKIYINGKLIDTIKVKELTGINF SKTITFEINKIPDTGLITSDSDNINMWIRDFYIFAKELDGKDINILFNSLQYTNVVKDYW GNDLRYNKEYYMVNIDYLNRYMYANSRQIVFNTRRNNNDFNEGYKIIIKRIRGNTNDT RVRGGDILYFDMTINNKAYNLFMKNETMYADNHSTEDIYAIGLREQTKDINDNIIFQIQ PMNNTYYYASQIFKSNFNGENISGICSIGTYRFRLGGDWYRHNYLVPTVKQGNYASLLE STSTHWGFVPVSE |
| SEQ ID NO:11 | DDSTCIKVKNNRLPYVADKDSISQEIFENKIITDETNVQNYSDKFSLDESILDGQVPINP EIVDPLLPNVNMEPLNLPGEEIVFYDDITKYVDYLNSYYYLESQKLSNNVENITLTTSVE EALGYSNKIYTFLPSLAEKVNKGVQAGLFLNWANEVVEDFTTNIMKKDTLDKISDVSVII PYIGPALNIGNSALRGNFNQAFATAGVAFLLEGFPEFTIPALGVFTFYSSIQEREKIIKTIE NCLEQRVKRWKDSYQWMVSNWLSRITTQFNHINYQMYDSLSYQADAIKAKIDLEYKK |

(continued)

| Table 5 Sequence Listing | |
|---|---|
| | YSGSDKENIKSQVENLKNSLDVKISEAMNNINKFIRECSVTYLFKNMLPKVIDELNKFDL RTKTELINLIDSHNIILVGEVDRLKAKVNESFENTMPFNIFSYTNNSLLKDIINEYFNSIN DSKILSLQNKKNALVDTSGYNAEVRVGDNVQLNTIYTNDFKLSSSGDKIIVNLNNNILY SAIYENSSVSFWIKISKDLTNSHNEYTIINSIEQNSGWKLCIRNGNIEWILQDVNRKYKS LIFDYSESLSHTGYTNKWFFVTITNNIMGYMKLYINGELKQSQKIEDLDEVKLDKTIVFG IDENIDENQMLWIRDFNIFSKELSNEDINIVYEGQILRNVIKDYWGNPLKFDTEYYIIND NYIDRYIAPESNVLVLVQYPDRSKLYTGNPITIKSVSDKNPYSRILNGDNIILHMLYNSRK YMIIRDTDTIYATQGGECSQNCVYALKLQSNLGNYGIGIFSIKNIVSKNKYCSQIFSSFR ENTMLLADIYKPWRFSFKNAYTPVAVTNYETKLLSTSSFWKFISRDPGWVE |
| SEQ ID NO:12 | KSICIEINNGELFFVASENSYNDDNINTPKEIDDTVTSNNNYENDLDQVILNFNSESAPG LSDEKLNLTIQNDAYIPKYDSNGTSDIEQHDVNELNVFFYLDAQKVPEGENNVNLTSSI DTALLEQPKIYTFFSSEFINNVNKPVQAALFVGWIQQVLVDFTTEANQKSTVDKIADISI VVPYIGLALNIGNEAQKGNFKDALELLGAGILLEFEPELLIPTILVFTIKSFLGSSDNKNKV IKAINNALKERDEKWKEVYSFIVSNWMTKINTQFNKRKEQMYQALQNQVNALKAIIES KYNSYTLEEKNELTNKYDIEQIENELNQKVSIAMNNIDRFLTESSISYLMKLINEVKINKL REYDENVKTYLLDYIIKHGSILGESQQELNSMVIDTLNNSIPFKLSSYTDDKILISYFNKF FKRIKSSSVLNMRYKNDKYVDTSGYDSNININGDVYKYPTNKNQFGIYNDKLSEVNISQ NDYIIYDNKYKNFSISFWVRIPNYDNKIVNVNNEYTIINCMRDNNSGWKVSLNHNEII WTLQDNSGINQKLAFNYGNANGISDYINKWIFVTITNDRLGDSKLYINGNLIDKKSILN LGNIHVSDNILFKIVNCSYTRYIGIRYFNIFDKELDETEIQTLYNNEPNANILKDFWGNY LLYDKEYYLLNVLKPNNFINRRTDSTLSINNIRSTILLANRLYSGIKVKIQRVNNSSTNDN LVRKNDQVYINFVASKTHLLPLYADTATTNKEKTIKISSSGNRFNQVVVMNSVGNCTMN FKNNNGNNIGLLGFKADTVVASTWYYTHMRDNTNSNGFFWNFISEEHGWQEK |

(continued)

| Table 5 Sequence Listing | |
|---|---|
| SEQ ID NO:13 | APPRLCIRVNNSELFFVASESSYNENDINTPKEIDDTTNLNNNYRNNLDEVILDYNSQTI PQISNRTLNTLVQDNSYVPRYDSNGTSEIEEYDVVDFNVFFYLHAQKVPEGETNISLTS SIDTALLEESKDIFFSSEFIDTINKPVNAALFIDWISKVIRDFTTEATQKSTVDKIADISLI VPYVGLALNIIIEAEKGNFEEAFELLGVGILLEFVPELTIPVILVFTIKSYIDSYENKNKAIK AINNSLIEREAKWKEIYSWIVSNWLTRINTQFNKRKEQMYQALQNQVDAIKTAIEYKY NNYTSDEKNRLESEYNINNIEEELNKKVSLAMKNIERFMTESSISYLMKLINEAKVGKLK KYDNHVKSDLLNYILDHRSILGEQTNELSDLVTSTLNSSIPFELSSYTNDKILIIYFNRLY KKIKDSSILDMRYENNKFIDISGYGSNISINGNVYIYSTNRNQFGIYNSRLSEVNIAQNN DIIYNSRYQNFSISFWVRIPKHYKPMNHNREYTIINCMGNNNSGWKISLRTVRDCEII WTLQDTSGNKENLIFRYEELNRISNYINKWIFVTITNNRLGNSRIYINGNLIVEKSISNL GDIHVSDNILFKIVGCDDETYVGIRYFKVFNTELDKTEIETLYSNEPDPSILKNYWGNYL LYNKKYYLFNLLRKDKYITLNSGILNINQQRGVTEGSVFLNYKLYEGVEVIIRKNGPIDIS NTDNFVRKNDLAYINVVDRGVEYRLYADTKSEKEKIIRTSNLNDSLGQIIVMDSIGNNC TMNFQNNNGSNIGLLGFHSNNLVASSWYYNNIRRNTSSNGCFWSSISKENGWKE |

(continued)

| Table 5 Sequence Listing | |
|---|---|
| SEQ ID NO:14 | SEQCIIVNNEDLFFIANKDSFSKDLAKAETIAYNTQNNTIENNFSIDQLILDNDLSSGIDL PNENTEPFTNFDDIDIPVYIKQSALKKIFVDGDSLFEYLHAQTFPSNIENLQLTNSLNDA LRNNNKVYTFFSTNLVEKANTVVGASLFVNWVKGVIDDFTSESTQKSTIDKVSDVSIII PYIGPALNVGNETAKENFKNAFEIGGAAILMEFIPELIVPIVGFFTLESYVGNKGHIIMTI SNALKKRDQKWTDMYGLIVSQWLSTVNTQFYTIKERMYNALNNQSQAIEKIIEDQYN RYSEEDKMNINIDFNDIDFKLNQSINLAINNIDDFINQCSISYLMNRMIPLAVKKLKDFD DNLKRDLLEYIDTNELYLLDEVNILKSKVNRHLKDSIPFDLSLYTKDTILIQVFNNYISNIS SNAILSLSYRGGRLIDSSGYGATMNVGSDVIFNDIGNGQFKLNNSENSNITAHQSKFVV YDSMFDNFSINFWVRTPKYNNNDIQTYLQNEYTIISCIKNDSGWKVSIKGNRIIWTLID VNAKSKSIFFEYSIKDNISDYINKWFSITITNDRLGNANIYINGSLKKSEKILNLDRINSS NDIDFKLINCTDTTKFVWIKDFNIFGRELNATEVSSLYWIQSSTNTLKDFWGNPLRYDT QYYLFNQGMQNIYIKYFSKASMGETAPRTNFNNAAINYQNLYLGLRFIIKKASNSRNIN NDNIVREGDYIYLNIDNISDESYRVYVLVNSKEIQTQLFLAPINDDPTFYDVLQIKKYYE KTTYNCQILCEKDTKTFGLFGIGKFVKDYGYVWDTYDNYFCISQWYLRRISENINKLRL GCNWQFIPVDEGWTE |

Botulinum toxin-related products have broad application prospects in the fields of treatment of clinical spastic diseases, glandular hypersecretion and neuropathic pains as well as cosmetic and wrinkle reduction. However, these products are currently administrated in a way of injection since they belong to macromolecules, and difficultly permeate through the skin to enter the site of action so as to exert the effect.

By being verified via a pharmacological experiment in mice, the transdermal composition of the present disclosure enables the permeation of the biological macromolecule to achieve the predetermined effect.

Preferably, based on the percentage of the entire transdermal composition, the effective amount of clostridial toxin is less than or equal to 0.02%, more preferably less than or equal to 0.015%, and even more preferably less than or equal to 0.012%;

In one example, the active substance comprises any one or more of a yeast extract, a plant extract and other substances that can exert any one or more effects of whitening, moisturizing, anti-aging, repairing and cell growth of the skin. Furthermore, in order to conduct mass differentiation of substances that may have the same effects as those of the active substance in the matrix component, it is also stipulated that the active substance is different from the components in the aforementioned matrix component (note: this description mainly makes distinguishing based on the function mainly achieved by the component, and is primarily intended to facilitate the description of component proportions, and is not a limitation: the matrix component cannot comprise the components that also have the same functions as those of the active substance. That is to say, the matrix component certainly contains a component that can function as the active substance at the same time. Only when it is mainly used as the matrix component, in order to distinguish the proportion description, the effect serving as the matrix component is emphasized. For example, when glycerin is used as a main component in the matrix component, glycerin also has the same effect as that of the active substance: the effect of moisturizing and lubricating on the skin. However, for the convenience of explanation, in this moment, a main function is to use glycerin as the component of the matrix component, even if glycerin also exerts the effect of the active substance, it is no longer classified as the active substance. This is only a formal compositional division for the sake of protection illustration).

Further, in a specific example, the active substance comprises any one or more of a yeast extract, sodium ascorbyl phosphate, *Hippophae rhamnoides* seed oil, any vitamin (e.g., vitamin E and niacinamide), retinyl palmitate, an aloe extract, allantoin, a glycyrrhiza glabra extract, a ginseng extract, a *Rheum palmatum* extract, a *Centella asiatica* extract, a tea extract and a *Hamamelis virginiana* extract. Of course, the active substance mentioned in this paragraph may also comprise the aforementioned biomolecule.

Vitamins are essential organic substances for the human body. Addition of vitamins and their derivatives has the effects of skin whitening, anti-aging and the like. Vitamin A promotes the proliferation of epidermal cells and increases the formation of dermal collagen and elastin. Vitamin E is a natural antioxidant that promotes metabolism and improves skin elasticity. Vitamin C has strong antioxidant properties, promoting collagen synthesis and inhibiting collagen breakdown. Vitamin C and vitamin E have the effect of synergistically scavenging free radicals. Niacinamide (vitamin B3) can reduce wrinkles by increasing collagen. Niacinamide also increases the activity of histone acetyltransferase in human fibroblasts while reducing the levels of histone acetyltransferase and histone H4 in senescent cells.

In addition to essential vitamins, a variety of natural plant active ingredients such as a tea extract, a ginseng extract, a *Centella asiatica* extract and a licorice extract have been shown to have anti-wrinkle, firming and whitening functions. These raw materials are currently widely used in anti-wrinkle, firming and whitening cosmetics.

However, in common cosmetics, the active ingredients in these raw materials have limited absorption on the skin surface, resulting in poor results. The transdermal composition of the present disclosure significantly promotes the transdermal absorption of active ingredients, and its practical effect has been demonstrated in a human efficacy experiment.

Table 6 and Table 7 are two examples of the components of the transdermal composition provided by the present disclosure.

| Table 6 | | |
|---|---|---|
| Name of composition | Category | In percentage by mass based on the total weight of the transdermal composition |
| Oleic acid | Oil phase | 1%-15% |
| Oleyl alcohol | | 10%-30% |
| Propylene glycol | | 5%-15% |
| Menthol | | 0.5%-4%, or 1%-2% |
| Laurocapram | | 1%-5%, or 2%-4%, or 2% |
| Any one or more of Polysorbate 80, Polysorbate 60, and Polysorbate 20 | Emulsifier | 30%-40% |
| Sorbitan oleate | | 10%-15% |
| Phenoxyethanol | Oil phase | 0.1%-1%, or 0.5% |
| Water | Aqueous phase | 10%-15% |
| Botulinum Toxin, type A | | 10 ng/ml-10 μg/ml |

| Table 7 | | |
| --- | --- | --- |
| Name of composition | Category | In percentage by mass based on the total weight of the transdermal composition |
| Oleic acid | Oil phase | 1%-15% |
| Oleyl alcohol | | 10%-30% |
| Propylene glycol | | 5%-15% |
| Menthol | | 0.5%-4%, or 1%-2% |
| Laurocapram | | 1%-5%, or 2%-4%, or 2% |
| Phenoxyethanol | | 0.1%-1%, or 0.5% |
| *Hippophae Rhamnoides* seed oil | | 0.5%-5% |
| Tocopherol (Vitamin E) | | 0.1%-1% |
| Retinyl palmitate | | 0.1%-0.2% |
| Any one or more of Polysorbate 80, Polysorbate 60, and Polysorbate 20 | Emulsifier | 30%-40% |
| Sorbitan oleate | | 10%-15% |
| Water | Aqueous phase | 10%-20% |
| Yeast extract | | 0.02%-0.2% |
| Sodium ascorbyl phophate | | 0.2%-2% |
| Nicotinamide | | 0.5%-5% |
| Aloe extract | | 0.2%-2% |
| Allantoin | | 0.1%-1 % |
| *Glycyrrhiza Glabra* extract | | 0.02%-0.2% |
| *Panax Ginseng* extract | | 0.02%-0.2% |
| *Rheum Palmatum* extract | | 0.02%-0.2% |
| *Centella Asiatica* extract | | 0.02%-0.2% |
| *Camellia Sinensis* leaf extract | | 0.02%-0.2% |
| *Hamamelis Virginiana* extract | | 0.02%-0.2% |

The transdermal composition of the present disclosure is used to achieve therapeutic, cosmetic and skin care effects. When in use, it can be transdermally applied through any one or more of applying, a transdermal instrument, patching and spraying.

The transdermal composition of the present disclosure can also be used to prepare a medicament or a product for achieving therapeutic, cosmetic and skin care effects.

The present disclosure further provides a preparation method of a transdermal composition as described above, comprising:

dissolving and mixing dissolved oil phase raw materials respectively, and then dissolving and mixing the obtained mixtures respectively to obtain a final oil phase mixture; and
adding aqueous phase raw materials directly or after dissolving into the final oil phase mixture to be mixed.

In an example, a transdermal composition having the following components is prepared:

oleic acid, oleyl alcohol, propylene glycol, menthol, laurocapram, sorbitan oleate, any one or more of polysorbate 80, polysorbate 60, and polysorbate 20, water and an effective amount of biomolecule. In this case, the preparation method comprises:
obtaining a first oil phase group: uniformly mixing a quantitative polysorbate 80, polysorbate 60 and sorbitan oleate;

then adding a quantitative oleyl alcohol, oleic acid, *Hippophae rhamnoides* seed oil, tocopherol, and retinyl palmitate to continue uniform mixing; then adding a quantitative laurocapram and phenoxyethanol to continue uniform mixing to obtain the first oil phase group;

obtaining a second oil phase group: mixing and fully dissolving a quantitative propylene glycol and menthol to obtain the second oil phase group;

obtaining a final oil phase mixture: uniformly mixing the first oil phase group and the second oil phase group to obtain the final oil phase mixture; adding an effective amount of the biomolecule to the final oil phase mixture, mixing uniformly, and supplementing water.

In an example, a transdermal composition having the following components is prepared:

oleic acid, oleyl alcohol, propylene glycol, menthol, laurocapram, sorbitan oleate, any one or more of polysorbate 80, polysorbate 60, and polysorbate 20, water, and effective amounts of yeast extract, sodium ascorbyl phosphate, *Hippophae rhamnoides* seed oil, optional vitamins, retinyl palmitate, aloe extract, allantoin, *Glycyrrhiza glabra* extract, ginseng extract, *Rheum palmatum* extract, *Centella asiatica* extract, tea extract, and witch hazel. In this case, the preparation method specifically comprises:

obtaining a first oil phase group: uniformly mixing a quantitative polysorbate 80, polysorbate 60, and sorbitan oleate; then adding a quantitative oleyl alcohol, oleic acid, *Hippophae rhamnoides* seed oil, tocopherol, and retinyl palmitate and continuing to mix uniformly; then adding a quantitative laurocapram and phenoxyethanol to continue uniform mixing to obtain the first oil phase group;

obtaining a second oil phase group: mixing and fully dissolving a quantitative propylene glycol and menthol to obtain the second oil phase group;

obtaining a final oil phase mixture: uniformly mixing the first oil phase group and the second oil phase group to obtain the final oil phase mixture;

separately adding a quantitative plant extract, yeast extract, and sodium ascorbyl phosphate, niacinamide, and allantoin separately dissolved in water to the final oil phase mixture in sequence, mixing evenly, and then supplementing water.

In addition, in the present disclosure, water is preferably a combination of any one or more of distilled water, drinking water, sterile water, ultrapure water and deionized water.

**Examples 1-6**

In Examples 1-6, transdermal compositions were prepared by using type A botulinum toxin as an active substance. The specific components are as shown in Table 8.

| Table 8 In percentage by mass based on the total weight of the transdermal composition | | | | | | |
|---|---|---|---|---|---|---|
| Name of composition | Example 1 | Example 2 | Example 3 | Example 4 | Example 5 | Example 6 |
| Oleic acid | 10.283% | 10.283% | 10.283% | 1.000% | 1.000% | 1.000% |
| Oleyl alcohol | 12.754% | 12.754% | 12.754% | 22.038% | 22.038% | 22.038% |
| Propylene glycol | 11.592% | 11.592% | 11.592% | 11.592% | 11.592% | 11.592% |
| Menthol | 1.000% | 1.000% | 1.000% | 1.000% | 1.000% | 1.000% |
| Laurocapram | 2.000% | 2.000% | 2.000% | 2.000% | 2.000% | 2.000% |
| Polysorbate 80 | 35.574% | 10.683% | 10.683% | 35.574% | 10.683% | 10.683% |
| Polysorbate 60 | - | - | 24.891% | - | - | 24.891% |
| Polysorbate 20 | - | 24.891% | - | - | 24.891% | - |
| Sorbitan oleate | 13.118% | 13.118% | 13.118% | 13.118% | 13.118% | 13.118% |
| Phenoxyethanol | 0.500% | 0.500% | 0.500% | 0.500% | 0.500% | 0.500% |
| Botulinum Toxin protein, type A | 0.001% | 0.001% | 0.001% | 0.001% | 0.001% | 0.001% |

(continued)

| Table 8 In percentage by mass based on the total weight of the transdermal composition | | | | | | |
|---|---|---|---|---|---|---|
| Name of composition | Example 1 | Example 2 | Example 3 | Example 4 | Example 5 | Example 6 |
| Purified water | 13.178% | 13.178% | 13.178% | 13.177% | 13.177% | 13.177% |
| "-"represents absence of addition | | | | | | |

Preparation method:

Step 1: according to a formulation, any one or more of quantitative polysorbate 80, polysorbate 60 and polysorbate 20, and sorbitan oleate were added into a container and stirred for 5 min; quantitative oleyl alcohol and oleic acid were further added into the container and stirred for 5 min; and quantitative laurocapram and phenoxyethanol were further added into the container and stirred evenly at room temperature (the rotation speed: 200-250 rpm, 5 min);
Step 2: according to the formulation, propylene glycol and menthol were separately weighed, mixed and heated to 60°C (for about 10 min) until being fully dissolved, and then the dissolved mixture was added into the solution obtained in Step 1, and the above materials were stirred evenly at room temperature (the rotation speed: 200-250 rpm, 5 min);
Step 3:10 mg of type A botulinum toxin was added into the solution prepared in Step 2, and the above materials were stirred at room temperature and mixed well (the rotation speed: 200-250 rpm, 5 min).
Step 4: purified water was added into the solution prepared in Step 3 for sufficient supplementation, and the above materials were stirred at room temperature and mixed well.

**Example 7**

Transdermal efficiency detection was conducted in this experiment.
The type A botulinum toxin added in Example 1 was replaced with a BSA protein. The prepared BSA protein emulsion was 0.62 mg/ml in concentration, and a transdermal experiment was conducted using a Franz diffusion cell method.

Experimental equipment and materials

1. Reagents

Phosphate buffer, protein standard samples, test samples, protein detection kit (ultrahigh sensitivity)

2. Consumables

Isolated hogskins for transdermal evaluation, centrifuge tubes, EP tubes, pipette tips, and pre-coated microplates

3. Equipment

TP-6 transdermal diffusion instrument (Tianjin Jingtuo Instrument Technology Co., Ltd.), multifunctional microplate reader (Thermo scientific, Varioskan Lux), medical refrigerator (Qingdao Haier Biomedical Co., Ltd.), biochemical incubator (Shanghai Yiheng Scientific Instrument Co., Ltd.), and pipette (Eppendorf AG).

I. Preparation before experiment:

(1) The hogskin required for the experiment was taken out from a refrigerator with - 20°C and thawed at 4°C, then washed with normal saline for later use.
(2) A supply chamber, a diffusion chamber (a receiving chamber for receiving a liquid from the supply chamber) and a stirrer were washed before the experiment.
(3) Pure water was added into a water tank to be flushed with a diffusion cell frame, and a temperature of 32°C and a rotation speed were set.

(4) After the temperature of the TP-6 transdermal diffusion instrument was raised to the set temperature, the hogskin was placed between the supply chamber and the diffusion chamber. The uniform stirring was conducted, so that the solution was kept at an isothermal state, and the seepage had a uniform concentration.

II. Experimental method:

(1) 2 experimental groups and 1 blank group were set.

(2) Addition of BSA protein test solution: 150 $\mu$l (0.62 mg/ml) of BSA protein emulsion test solution prepared in Example 1, 150 $\mu$l (1 mg/ml) of BSA protein test solution dissolved in PBS were respectively added into the first 2 supply chambers, 3 ml of PBS was added into the blank group.

(3) The receiving chamber was filled with PBS, 4 1.5 ml EP tubes were taken, 400 $\mu$l of liquid in the receiving chamber was taken from the sampling port of the receiving chamber every 1 h for subsequent OD detection, and sampling time and experiment group numbers were labeled, with total sampling for 6 h. After sampling, each receiving chamber must be supplemented with 400 $\mu$l of PBS to maintain the volume of the receiving chamber unchanged.

(4) All the collected samples were placed in a refrigerator at 4°C for later use.

III. ELISA experiment detection:

1. Reagent preparation:

a. All reagents were equilibrated to room temperature (18-25°C) before use. Follow the instrument of the microplate reader, a detection wavelength was set as 450 nm and the microplate reader was preheated for 15 min before reading the plate.

b. Washing buffer: 20 ml of concentrated washing buffer was diluted with 580 ml of deionized water to prepare 600 ml of washing buffer.

c. Standard working solution: first, 1000 g of standard sample was centrifuged for 1 min, and 2 ml of standard sample diluent was added so that the standard sample was mixed with the standard sample diluent. 300 $\mu$l of 20000 ng/ml standard diluent was sucked into the first tube to be mixed well to obtain a 6666.7 ng/ml working solution. 300 $\mu$l of the solution was transferred from the front tube to the rear tube. Before the next transfer, each tube was thoroughly mixed well. The standard sample was diluted at 6 points, 20000 ng/ml, 6666.7 ng/ml, 2222.2 ng/ml, 740.7 ng/ml, 246.9 ng/ml and 0 ng/ml, respectively.

d. Detection reagent A working solution: the required amount (100 $\mu$l/well) was calculated before the experiment. In the preparation works, an amount that was 100-200 $\mu$l more than the calculated amount should be prepared. The stock solution tube was slightly centrifuged before use, and a 100 $\times$ concentrated detection reagent A was diluted to a 1$\times$ working solution A with a detection diluent A (such as: 10 $\mu$l of detection reagent A + 990 $\mu$l of detection reagent A diluent).

e. Detection reagent B working solution: the required amount (100 $\mu$l/well) was calculated before the experiment. In the preparation works, an amount that was 100-200 $\mu$l more than the calculated amount should be prepared. The stock solution tube was slightly centrifuged before use, and a 100 $\times$ concentrated detection reagent B was diluted to a 1 $\times$ working solution B with a detection diluent B (such as: 10 $\mu$l of detection reagent B + 990 $\mu$l of detection reagent B diluent).

2. Experimental method

(1) Standard sample wells, sample wells and blank wells were respectively set. 6 different concentrations of standard samples (containing zero wells, 50 $\mu$L/well) were added in turn, and 50 $\mu$L of samples to be tested (samples collected from the sampling port of the receiving chamber) were added into other wells, and then the detection reagent A working solution (50 $\mu$L/well) was immediately added, gently shaken and mixed well. The well plate was covered with a plate sealing film provided by the kit, and the samples were incubated at 37°C for 60 min.

(2) The liquids in all wells were discarded, 350 $\mu$L of washing buffer was added into each well to soak the samples for 60 s and then the liquid in each well was poured out and patted dry on clean absorbent paper. The washing step was repeated for 3 times in total.

(3) 100 μL of detection reagent B working solution was added in each well. The well plate was covered with the plate sealing film, and the samples were incubated at 37°C for 30 min.

(4) The liquid in each well was discarded, and the washing process in Step 2 was repeated 5 times.

(5) 90 μL of TMB reagent was added into each well, and the well plate was covered with a new plate sealing film, and the samples were incubated at 37°C for 10-20 min in the dark.

(6) 50 μL of stop solution was added into each well (the order was consistent with the order of adding a color developer), and gently shaken and mixed well.

(7) The well bottom of the ELISA plate was ensured to have no bubbles and water mist, and the absorbance OD value of each well was then immediately measured at 450 nm, and the reading of the microplate reader was recorded.

IV. Test data and conclusions

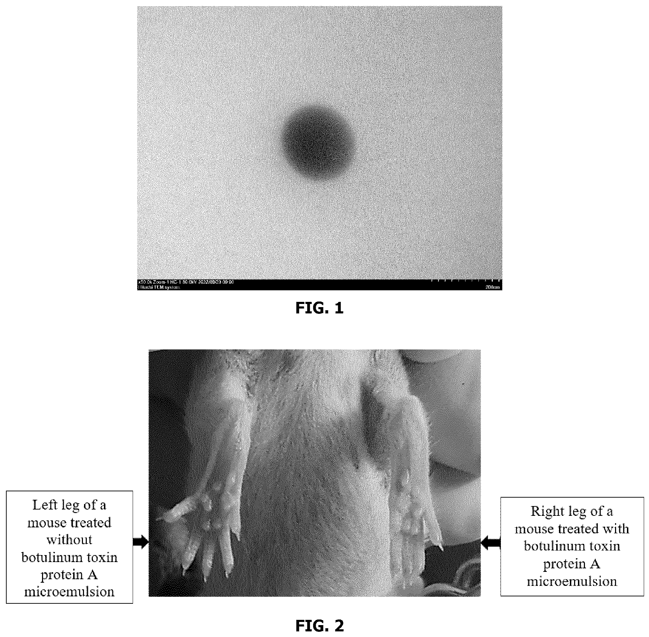

(1) The standard curve of BSA standard sample fit based on four parameters is shown in FIG. 1.

Plotting of standard curve:

The OD value data of the detected standard protein was processed:

The data was fitted with four parameters to obtain a standard curve, and the four-parameter fitting formula is as follows:

$$y = bottom + \frac{top - bottom}{1 + \left(\frac{IC50}{x}\right)^{HillSlope}}$$

The specific standard curve is shown in FIG. 1.

(2) Sample detection results

Table 9 shows BSA concentrations of different test samples permeating through the skin at different time periods calculated based on the standard curve.

| Table 9 | | |
|---|---|---|
| Time (h) | BSA emulsion prepared in Example 1 (ng/ml) | BSA dissolved in PBS (ng/ml) |
| 0 | 0 | 0 |
| 1.5 | 95.2 | 68 |
| 3 | 147.1 | 108.7 |
| 6 | 129.1 | 110 |
| 12 | 142.7 | 125.4 |
| 24 | 134.1 | 114.7 |

According to the results, transdermal calculation was performed:

(1) In Comparative example 2, a BSA emulsion protein test solution was prepared. After 24 h, the concentration of BSA in the receiving chamber was 147.1 ng/ml, and the volume of BSA in the receiving chamber was 15 ml. The concentration of BSA in the supply chamber was 0.62 mg/ml, and the volume of BSA added into the supply chamber was 150 μl. And therefore, the transdermal efficiency of the BSA emulsion prepared in Example 5 was calculated to be 2.37%.

(2) After 24 h, the concentration of the BSA protein test solution dissolved in PBS in the receiving chamber was 125.4 ng/ml, the volume of BSA was 15 ml, while the concentration of BSA in the supply chamber was 1 mg/ml, and the volume of BSA added into the supply chamber was 150 μl. And therefore, the transdermal efficiency of PBS was calculated to be 1.25%.

It can be seen that compared with control, the transdermal effect in Example 1 was very good.

**Example 8**

The transdermal compositions prepared in Examples 1-6 were subjected to efficacy verification experiments in mice.

17-19 g of SPF grade CD-1 (ICR) mice were used. After the mice were anesthetized with tribromethanol, the hairs on the skin on the right hind leg of the mouse were shaved. Before application each time, an applying site was washed with a medical cotton swab dipped in 10% alcohol. After drying in the air, the medicine was applied to the applying site. 200 μl of prepared transdermal composition was applied onto the skin on the right hind leg of the mouse once a day for 5 consecutive days. The left hind legs of the mouse were not applied using the transdermal composition as control group.

It is found in the experiment results that, after 3-5 days of applying, the toes of the right hind leg of the mouse would have a phenotype of syndactyly of the toes (2 toes or 3 toes syndactyly), and the phenotype of the 3 toes syndactyly is shown in FIG. 2, while control group had no this phenotype.

However, according to a literature report ("An Alternative In Vivo Method to Refine the Mouse Bioassay for Botulinum Toxin Detection", Temeri D Wilder-Kofie et al., Vol. 61, No. 3, June 2011), after the gastrocnemius muscle of the mouse was injected with botulinum toxin protein A, the phenotype of syndactyly of the toes occurred. The transdermal composition of the present disclosure was applied to the skin of the mouse to achieve the phenotype of syndactyly of the toes after the injection of botulinum toxin protein A. It can be seen that the transdermal composition of the present disclosure achieved the effective transdermal administration of the type A botulinum toxin, that is, the transdermal composition of the present disclosure can allow the effective transdermal administration of macromolecules so as to achieve the predetermined effect.

**Example 9**

In this example, a transdermal composition was prepared according to the formulation as shown in Table 10.

| Table 10 In percentage by mass based on the total weight of the transdermal composition | |
| --- | --- |
| Oleic acid | 1.000% |
| Oleyl alcohol | 21.110% |
| Propylene glycol | 11.120% |
| Menthol | 0.500% |
| Laurocapram | 2.000% |
| Polysorbate 80 | 10.250% |
| Polysorbate 60 | - |
| Polysorbate 20 | 23.880% |
| Sorbitan oleate | 10.000% |
| Phenoxyethanol | 0.500% |
| Purified water | 16.500% |
| Yeast extract | 0.020% |
| Sodium ascorbyl phophate | 1.000% |
| *Hippophae Rhamnoides* seed oil | 0.500% |
| Tocopherol (Vitamin E) | 0.500% |
| Nicotinamide | 0.500% |
| Retinyl palmitate | 0.200% |
| Aloe extract | 0.200% |
| Allantoin | 0.100% |

(continued)

| Table 10 | |
|---|---|
| In percentage by mass based on the total weight of the transdermal composition | |
| *Glycyrrhiza Glabra* extract | 0.020% |
| *Panax Ginseng* extract | 0.020% |
| *Rheum Palmatum* extract | 0.020% |
| *Centella Asiatica* extract | 0.020% |
| *Camellia Sinensis* leaf extract | 0.020% |
| *Hamamelis Virginiana* extract | 0.020% |

Preparation method:

Step 1: according to the formulation, quantitative polysorbate-80, polysorbate-20, and sorbitan oleate were added into a container and stirred for 5 min; quantitative oleyl alcohol, oleic acid, sea buckthorn seed oil, tocopherol and retinol palmitate were added into the container and stirred for 5 min; and quantitative laurazole ketone and phenoxyethanol were further added into the container and stirred evenly at room temperature (the rotation speed: 200-250 rpm, 5 min);
Step 2: according to the formulation, propylene glycol and menthol were weighed separately, mixed, heated to 60°C (about 10 min) until fully dissolved, then the dissolved mixture was added into the solution obtained in Step 1, and stirred evenly at room temperature (the rotation speed: 200-250 rpm, 5 min);
Step 3: according to the formulation, a quantitative plant extracts (tea extract, ginseng extract, Centella asiatica extract, aloe extract, Glycyrrhiza glabra extract, Rheum palmatum extract, Hamamelis virginiana extract and yeast extract) were added into the solution prepared in step 2 and stirred at room temperature (the rotation speed: 200-250 rpm, 5 min);
Step 4: according to the formulation, a quantitative sodium ascorbic acid phosphate was dissolved with water and then added into the solution obtained in Step 3, and the above materials were stirred evenly at room temperature (the rotation speed: 200-250 rpm);
Step 5: according to the formulation, a quantitative amount of nicotinamide was added, dissolved with water and heated to 60°C until fully dissolved, then the dissolved mixture was added into the solution obtained in Step 4, and stirred evenly at room temperature (the rotation speed: 200-250 rpm);
Step 6: according to the formulation, a quantitative amount of allantoin was dissolved with water and heated to 80°C until fully dissolved, and then the dissolved mixture was added into the solution obtained in Step 5, and stirred evenly at room temperature (the rotation speed: 200-250 rpm);
Step 7: according to the formulation, purified water was added into the solution prepared in Step 6 for sufficient supplementation, and the above materials were stirred and evenly mixed at room temperature.

**Example 10**

The efficacy of the transdermal composition prepared in Example 9 in the human body was detected.
According to the prescribed subject standards, this example includes 33 subjects in total with final completion of 33 subjects. The ages of the 33 subjects are in a range of 35-55 years old, with an average age of 45.27 years old. 4 of the 33 subjects were male, and 29 of the 33 subjects were female. (See Table 10 for details).

Recruitment standards of subjects:

1) healthy men and women, aged 30-55, with obvious crow's feet, nasolabial folds, and forehead wrinkles;
2) be able to cooperate well with experimenters and maintain a regular life during the study;
3) be able to read and understand all the contents of the informed consent form and voluntarily sign the informed consent form;
4) agree not to use any cosmetics, drugs, or health products that may affect the results during the trial; and
5) other relevant standards.

Subject exclusion standards:

1) subjects who use antihistamine drugs in the past week or try immunosuppressants within the past month;
2) subjects who have applied any anti-inflammatory drugs onto the test site within the past two months;
3) subjects who have had a history of skin diseases (such as psoriasis, eczema, psoriasis and skin cancer);
4) patients with insulin-dependent diabetes;
5) patients with asthma or other chronic respiratory diseases who are receiving treatment;
6) patients who receive anti-cancer chemotherapy within the past 6 months;
7) patients with immunodeficiency or autoimmune diseases;
8) lactating or pregnant women;
9) patients with bilateral mastectomy and bilateral axillary lymph node removal;
10) the deciders of the skin sites to be tested whose test results are affected by scars, pigment, atrophy, bright erythema, uneven skin tone, folliculitis or other defects;
11) patients with any other health problems or chronic diseases;
12) subjects applied with retinoids A, hydroxy acid, salicylic acid, hydroquinone within the past 3 months, or prescription drugs (antibiotics, retinoids A, hydroxy acid and steroids) within the past 6 months, and oral contraceptive drugs (if you have been taking the same contraceptives for the past 6 months, you can continue to take them); and
13) experts or professionals believe that the evaluation results are affected due to other iatrogenic reasons.

| Table 11 Details of the subjects | | | |
|---|---|---|---|
| Subject No. | Code | Age | Gender |
| 001 | ZZQ | 41 | Female |
| 002 | GDD | 38 | Female |
| 003 | CYH | 40 | Female |
| 004 | HKH | 46 | Female |
| 005 | YHM | 46 | Female |
| 006 | WXD | 35 | Male |
| 007 | LXM | 50 | Female |
| 008 | HCR | 45 | Female |
| 009 | LGQ | 55 | Female |
| 010 | LR | 45 | Female |
| 011 | LXH | 45 | Female |
| 012 | ZCH | 41 | Female |
| 013 | YL | 41 | Female |
| 014 | MWJ | 53 | Female |
| 015 | OYXW | 41 | Male |
| 016 | QZQ | 48 | Female |
| 017 | WXT | 48 | Female |
| 018 | FSF | 52 | Female |
| 019 | WXW | 54 | Female |
| 020 | ZH | 48 | Female |

| Table 11 Details of the subjects | | | |
|---|---|---|---|
| Subject No. | Code | Age | Gender |
| 021 | SYQ | 45 | Female |
| 022 | JPY | 52 | Female |
| 023 | GGH | 49 | Female |
| 024 | ZXH | 55 | Female |
| 025 | ZL | 42 | Male |
| 026 | YWJ | 41 | Male |
| 027 | XLL | 43 | Female |
| 028 | CF | 50 | Female |
| 029 | ZBW | 40 | Female |
| 030 | CXY | 38 | Female |
| 031 | LY | 39 | Female |
| 032 | LD | 41 | Female |
| 033 | ZMH | 47 | Female |

**1. Method of use**

Before going to bed every day, facial oil was washed thoroughly (such as soap or makeup remover). If you want to get the best absorption effect, it was recommended to wipe the face with a cleaning wipe to completely remove the oil residue. After the skin was dried, sites where wrinkles need to be removed are determined in front of a mirror.

A sample lid was opened and the bottom of the bottle was pressed until a liquid was pushed out around the balls. Roll back and forth with the balls 5 times until it was evenly applied. After applying, wait until the skin gradually absorbs, and do not wipe the skin during the period.

**2. Frequency of use:**

Use once every other day

**3. Test purpose**

The moisturizing, repairing, anti-wrinkle and firming effects of test samples were evaluated.

**4. Test environment**

Temperature: 21 ± 1°C
Humidity: 50 ± 10%RH

**5. Test method**

**5.1 Method reference**

1) QB/T 4256-2011 Guidelines for evaluating cosmetic moisturizing effects;
2) T/ZHCA 003-2018 Test method for cosmetics to affect transepidermal moisture loss;
3) T/ZHCA 006-2019 Cosmetic anti-wrinkle effect testing method;
4) T/TDCA 003-2021 Cosmetic firming effect testing method; and
5) Cosmetic safety technical specifications (2015 edition).

**5.2 Testing process**

**DO (refers to the Day 1 of the subject's visit):**

The information of the subjects were registered, and then laboratory technician screened out qualified subjects with obvious wrinkles (crow's feet level ≥2, nasolabial fold level ≥1, and headline fold level ≥1). After completing the inclusion and exclusion standards, the subject signed an informed consent form.

2) Subjects cleaned their faces with designated cleansing samples and sat in a laboratory with a temperature of 21 ± 1°C and 50 ± 10% RH for 30 min;

3) The subject's skin basic value was tested: Corneometer was used to test the skin's cuticle moisture content, Tewameter was used to test the skin's transepider moisture loss rate, Cutometer was used to test the skin's elasticity R2 value and firmness F4 value, and experts evaluated facial fine line, nasolabial fold, Chuan word pattern, forehead wrinkle, and lip corner wrinkle.

**D14 (refers to the subject's visit after 14 days using the sample):**

1) Subjects cleaned their faces with designated cleansing samples and sat in a laboratory with a temperature of 21 ± 1°C and 50 ± 10% RH for 30 min;
2) The data value for the subject's skin on day 14 was tested: Corneometer was used to test the skin's cuticle moisture content, Tewameter was used to test the skin's transepider moisture loss rate, Cutometer was used to test the skin's elasticity R2 value and firmness F4 value, and experts evaluated facial fine line, nasolabial fold, Chuan word pattern, forehead wrinkle, and lip corner wrinkle;

3) Subjects filled out the questionnaire.

**D28 (refers to the subject's visit after 28 days using the sample):**

1) Subjects cleaned their faces with designated cleansing samples and sat in a laboratory with a temperature of 21 $\pm$ 1°C and 50 $\pm$ 10% RH for 30 min;

2) The data value for the subject's skin on day 28 was tested: Corneometer was used to test the skin's cuticle moisture content, Tewameter was used to test the skin's transepider moisture loss rate, Cutometer was used to test the skin's elasticity R2 value and firmness F4 value, and experts evaluated facial fine line, nasolabial fold, Chuan word pattern, forehead wrinkle, and lip corner wrinkle.

3) Subjects filled out the questionnaire, and then the usage log was recycled.

**5.3 Test items (Table 12)**

| Table 12 | | | | | |
|---|---|---|---|---|---|
| No. | Test items | Test instrument/method | Test time point | Test site | Parameter Description |
| 1. | Water content of the skin's stratum corneum | Corneometer CM825 | D0/D14/ D28 | Cheek | The instrument is based on the dielectric constant of water (81) and other substances (<7) The variation is quite large. According to the different water content, the appropriate shape of the measuring capacitor will change with the change of the capacitance of the skin, and the capacitance of the skin is within the measurement range. , so that the moisture content of the skin can be measured. The larger the measurement value, the higher the moisture content of the skin's stratum corneum |
| 2. | Transepidermal water loss | Tewameter™ Hex | D0/D14/ D28 | Cheek | The Tewameter TM Hex probe contains 15 pairs of relative humidity and temperature sensors. The probe is like a camera. , the distribution of relative humidity and temperature in the cavity can be seen through a large amount of data measured each time. The smaller the measurement value, the longer the unit time and unit cross-sectional area. The less water loss from the epidermis, the better the skin barrier. |

(continued)

| No. | Test items | Test instrument/method | Test time point | Test site | Parameter Description |
|---|---|---|---|---|---|
| Table 12 | | | | | |
| 4. | Skin elasti-city and firm-ness | Cutometer dual MPA580 | D0/D14/D28 | Cheek | The testing principle of this instrument is based on the principles of suction and stretching. It generates a negative pressure on the surface of the skin being tested to suck the skin into a specific test probe. The depth of the skin sucked into the test probe is measured by a noncontact optical testing system. The smaller the F4 measurement value, the better the skin firmness The larger the R2 measurement value, the better the skin elasticity |
| 5. | Area ratio of under-eye wrinkles | IPP | D0/D14/D28 | Under-eye | The smaller the measurement value, the smaller the area of under-eye wrinkles |
| 6. | Expert eva-luation | Visual as-sessment | D0/D14/D28 | Full face | Visual assessment of wrinkles on the subjects' skin The smaller the evaluation value, the fewer fine lines on the face. The smaller the evaluation value, the fewer nasolabial folds. The smaller the evaluation value, the fewer the Chuan word patterns. The smaller the evaluation value, the fewer forehead wrinkles. The smaller the evaluation value, the fewer lip corner wrinkles. |
| 7. | Self assess-ment | Evaluation questionn aire | D14/D2 8 | Full face | The subjects evaluated their experience with the samples and calculated the positive review rate. |

## 6. Data statistical method

### 6.1 Descriptive Statistics

Instrument measurement: mean, standard error, maximum, minimum;
Subject self-assessment: proportion of identification degree.

### 6.2 Difference analysis

SPSS statistical analysis software was used to perform statistical analysis of data. The measurement data were expressed as: mean $\pm$ standard error, and normal distribution test was performed, which meets the requirements of normal distribution. The paired t test was used for comparison before and after oneself, otherwise two related samples were rank sum tests; two related samples were rank sum tests before and after the grade data were used; independent sample t test or rank sum test was used for comparison between the test products and the control group. All statistical analyses above were two-tailed tests, with a significance level of $\alpha$=0.05.

## 7. Test results

**7.1 Completion status of subject**

This example includes 33 subjects in total with final completion of 33 subjects. The ages of the 33 subjects are in a range of 35-55 years old, with an average age of 45.27 years old. 4 of the 33 subjects were male, and 29 of the 33 subjects were female, See Table 10 for details.

| Table 3 Test results of trial in human | | | | | | | |
|---|---|---|---|---|---|---|---|
| Test time | Subject no. | Status of adverse reaction of skin | | | | | |
| | | 0 | 1 | 2 | 3 | 4 |
| 14 days after using sample | 33 | 33 | 0 | 0 | 0 | 0 |
| 28 days after using sample | 33 | 33 | 0 | 0 | 0 | 0 |

**7.2 Feedback on adverse reactions during use**

| Table 14 Grading standard for adverse reaction in skin | |
|---|---|
| Grade | Skin reaction |
| 0 | No reaction |
| 1 | Weak erythema |
| 2 | Erythema, infiltration, papule |
| 3 | Erythema, edema, papule, pomphus |
| 4 | Erythema, edema, blister |
| Note: Refer to the grading standards for adverse reactions of skin in human trials stipulated in the 2015 edition of the "Safety and Technical Standards for Cosmetics" | |

**Results description: after the sample was used for 14 days, the skin reactions in the observation area were all grade 0, and no adverse reactions occurred in the skin;**
**after the sample was used for 28 days, the skin reactions in the observation area were grade 0 and no adverse reactions occurred in the skin.**

**7.3 Descriptive and statistical analysis of the test results of moisture content in the skin stratum corneum (the larger the measured value, the higher the moisture content in the skin stratum corneum)**

| Table 15 Descriptive analysis of the test results of moisture content in the skin stratum corneum | | | | | |
|---|---|---|---|---|---|
| Test time | Moisture content in the skin stratum corneum (a.u.) | | | Improvement rate of the | P value of comparison |
| | Maximum | Minimum | Mean $\pm$ standard error | moisture content in the skin stratum corneum (%) | with that before use |
| Before use | 46 | 19.27 | $35.89\pm1.28$ | / | / |
| Using the sample for 14 days | 57.07 | 27.43 | $43.51\pm1.22$ | 21.23 | P<0.001 |
| Using the sample for 28 days | 61.53 | 37.47 | $50.74\pm1.10$ | 41.38 | P<0.001 |
| Note: improvement rate (%)=(measured value after a sample is used for N days - measured value before use)/-measured value before use*100% | | | | | |

| Table 16 Statistical analysis of the test results of moisture content in the skin stratum corneum | | | | | | |
|---|---|---|---|---|---|---|
| Normality test before and after using the sample | | | Difference test before and after using the sample | | | |
| Statistical item | P | Results | Test Method | Criteria | P | Results |
| Before use and using the sample for 14 days | P=0.044 | Non-normal distribution | Wilcoxon signed-rank test | P<0.05 | P<0.001 | Significant difference |
| Before use and using the sample for 28 days | P=0.007 | Non-normal distribution | Wilcoxon signed-rank test | P<0.05 | P<0.001 | Significant difference |

Results description:

Compared with the situation before use, after the sample was used for 14 days, the improvement rate of moisture content in the skin stratum corneum was 21.23%, with a significant difference; and compared with the situation before use, after the sample was used for 28 days, the improvement rate of moisture content in the skin stratum corneum was 41.38%, with a significant difference.

7.4 Descriptive and statistical analysis of the test results of skin the transepidermal water loss rate (the smaller the measured value, the less transepidermal water loss amount per unit time and per unit cross-sectional area, which indicates the better the skin barrier)

| Table 17 Descriptive analysis of the test results of skin transepidermal water loss rate | | | | | |
|---|---|---|---|---|---|
| Test time | Skin transepidermal water loss rate (g/h/m$^2$) | | | Improvement rate of the skin transepidermal water loss rate (%) | P value of comparis on with that before use |
| | Maximum | Minimum | Mean ± standard error | | |
| Before use | 31.19 | 16.49 | 23.38±0.62 | / | / |
| Using the sample for 14 days | 28.39 | 13.93 | 19.68±0.61 | 15.84 | P<0.001 |
| Using the sample for 28 days | 26.9 | 12.1 | 16.67±0.57 | 28.73 | P<0.001 |
| Note: improvement rate (%)=(measured value before use-measured value after a sample is used for N days)/measured value before use*100% | | | | | |

| Table 18 Test results of skin transepidermal water loss rate | | | | | | |
|---|---|---|---|---|---|---|
| Normality test before and after using the sample | | | Difference test before and after using the sample | | | |
| Statistical item | P | Results | Test Method | Criteria | P | Results |
| Before use and using the sample for 14 days | P=0.010 | Non-normal distribution | Wilcoxon signed-rank test | P<0.05 | P<0.001 | Significant difference |
| Before use and using the sample for 28 days | P≥0.05 | Normal distribution | Paired *t*-test | P<0.05 | P<0.001 | Significant difference |

Results description:

compared with the situation before use, after the sample was used for 14 days, the improvement rate of transepidermal water loss rate in the skin was 15.84%, with a significant difference; and

compared with the situation before use, after the sample was used for 28 days, the improvement rate of transepidermal water loss rate in the skin was 28.73%, with a significant difference.

**7.5 Descriptive and statistical analysis of test results of the skin elasticity R2 value (the larger the measured value, the better the skin elasticity)**

| Table 19 Descriptive analysis of the test results of the skin elasticity R2 value | | | | | |
|---|---|---|---|---|---|
| Test time | Skin elasticity R2 value | | | Improvement rate of the R2 value of skin elasticity (%) | P value of comparison with that before use |
| | Maximum | Minimum | Mean ± standard error | | |
| Before use | 0.54 | 0.41 | 0.48±0.01 | / | / |
| Using the sample for 14 days | 0.62 | 0.47 | 0.53±0.01 | 11.02 | P<0.001 |
| Using the sample for 28 days | 0.68 | 0.49 | 0.58±0.01 | 20.13 | P<0.001 |

Note: improvement rate (%)=(measured value after a sample is used for N days - measured value before use)/measured value before use*100%

| Table 20 Statistical analysis of the test results of skin elasticity R2 | | | | | | |
|---|---|---|---|---|---|---|
| Normality test before and after using the sample | | | Difference test before and after using the sample | | | |
| Statistical item | P | Results | Test Method | Criteria | P | Results |
| Before use and using the sample for 14 days | P≥0.05 | Normal distribution | Paired *t*-test | P<0.05 | P<0.001 | Significant difference |
| Before use and using the sample for 28 days | P≥0.05 | Normal distribution | Paired *t*-test | P<0.05 | P<0.001 | Significant difference |

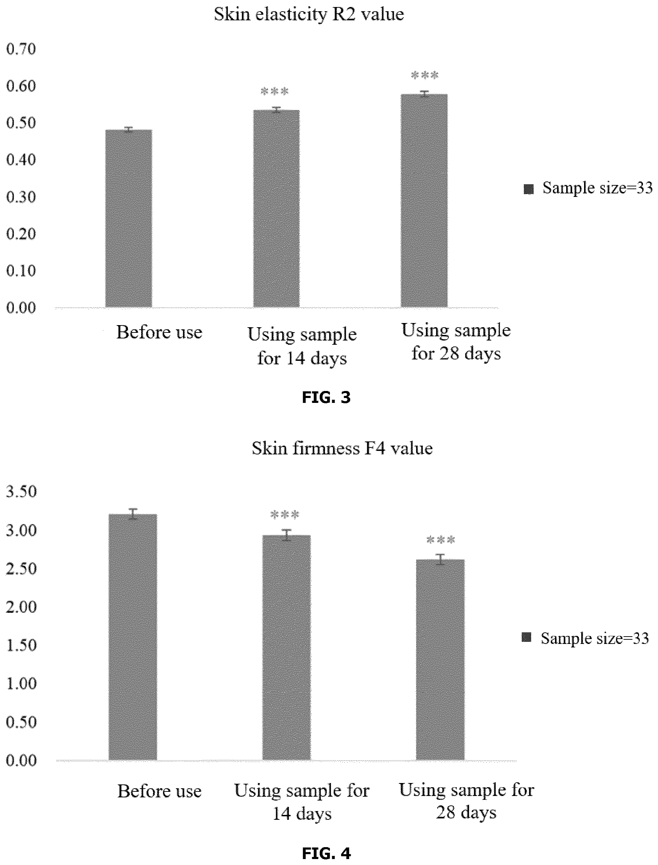

FIG. 3 is a comparison chart of skin elasticity R2 values before and after the transdermal composition prepared in Example 9 is used (the larger the R2 measurement value, the better the skin elasticity): significance marking method: "n.s" indicates no significant difference, P ≥ 0.05; "*" indicates a significant difference, 0.01 ≤ P < 0.05; "**", 0.001 ≤ P < 0.01; "***", P < 0.001.

The results showed that compared with the situation before use, the skin elasticity R2 values were significantly improved after 14 days and 28 days of use (P<0.05), with improvement rates of 11.02% and 20.13% respectively.

**7.6 Descriptive and statistical analysis of test results of skin firmness F4 value (the smaller the measured value, the better the skin firmness)**

| Table 21 Descriptive analysis of the test results of the skin firmness F4 value | | | | | |
|---|---|---|---|---|---|
| Test time | Skin firmness F4 value | | | Improvement rate of the skin firmness (%) | P value of comparison with that before use |
| | Maximum | Minimum | Mean ± standard error | | |
| Before use | 4.04 | 2.45 | 3.21±0.0 6 | / | / |
| Using the sample for 14 days | 3.61 | 2.06 | 2.94±0.0 7 | 8.45 | P<0.001 |

(continued)

| Table 21 Descriptive analysis of the test results of the skin firmness F4 value | | | | | |
|---|---|---|---|---|---|
| Test time | Skin firmness F4 value | | | Improvement rate of the skin firmness (%) | P value of comparison with that before use |
| | Maximum | Minimum | Mean ± standard error | | |
| Using the sample for 28 days | 3.38 | 1.93 | 2.62±0.0 7 | 18.35 | P<0.001 |
| Note: improvement rate (%)=(measured value before use-measured value after a sample is used for N days)/measured value before use*100% | | | | | |

| Table 22 Statistical analysis of the test results of the skin firmness F4 value | | | | | | |
|---|---|---|---|---|---|---|
| Normality test before and after using the sample | | | Difference test before and after using the sample | | | |
| Statistical item | P | Results | Test Method | Criteria | P | Results |
| Before use and using the sample for 14 days | P≥0.05 | Normal distribution | Paired *t*-test | P<0.05 | P<0.001 | Significant difference |
| Before use and using the sample for 28 days | P≥0.05 | Normal distribution | Paired *t*-test | P<0.05 | P<0.001 | Significant difference |

FIG. 4 is a comparison chart of skin firmness F4 values before and after transdermal composition prepared in Example 9 is used (the smaller the F4 value, the better the skin firmness): significance marking method: "n.s" indicates no significant difference, $P \geq 0.05$; "*" indicates a significant difference, $0.01 \leq P < 0.05$; "**", $0.001 \leq P < 0.01$; "***", $P < 0.001$.

The results showed that compared with the situation before use, the skin firmness F4 values were significantly reduced after 14 and 28 days of use (P<0.05), with improvement rates of 8.45% and 18.35%, respectively.

**7.7 Descriptive and statistical analysis of the area proportion of under-eye wrinkle (the smaller the measured value, the smaller the area proportion of under-eye wrinkle)**

| Table 23 Descriptive analysis of the test results of the area proportion of under-eye wrinkle | | | | | |
|---|---|---|---|---|---|
| Test time | Area proportion of under-eye wrinkle | | | Improvement rate of the area | P value of comparison |
| | Maximum | Minimum | Mean ± standard error | proportion of under-eye wrinkle (%) | with that before use |
| Before use | 0.13 | 0.02 | 0.05±0.01 | / | / |
| Using the sample for 14 days | 0.13 | 0.02 | 0.05±0.01 | 2.66 | P≥0.05 |
| Using the sample for 28 days | 0.12 | 0.02 | 0.05±0.00 | 8.46 | P=0.020 |
| Note: improvement rate (%)=(measured value before use-measured value after a sample is used for N days)/measured value before use*100% | | | | | |

| Table 24 | | | | | | |
|---|---|---|---|---|---|---|
| Normality test of the area of under-eye wrinkle before and after usage | | | Difference test of the area of under-eye wrinkle before and after usage | | | |
| Statistical item | P | Results | Test Method | Criteria | P | Results |
| Before use and using the sample for 14 days | P<0.001 | Non-normal distribution | Wilcoxon signed-rank test | P<0.05 | P≥0.05 | No significant difference |
| Before use and using the sample for 28 days | P=0.038 | Normal distribution | Wilcoxon signed-rank test | P<0.05 | P=0.020 | Significant difference |

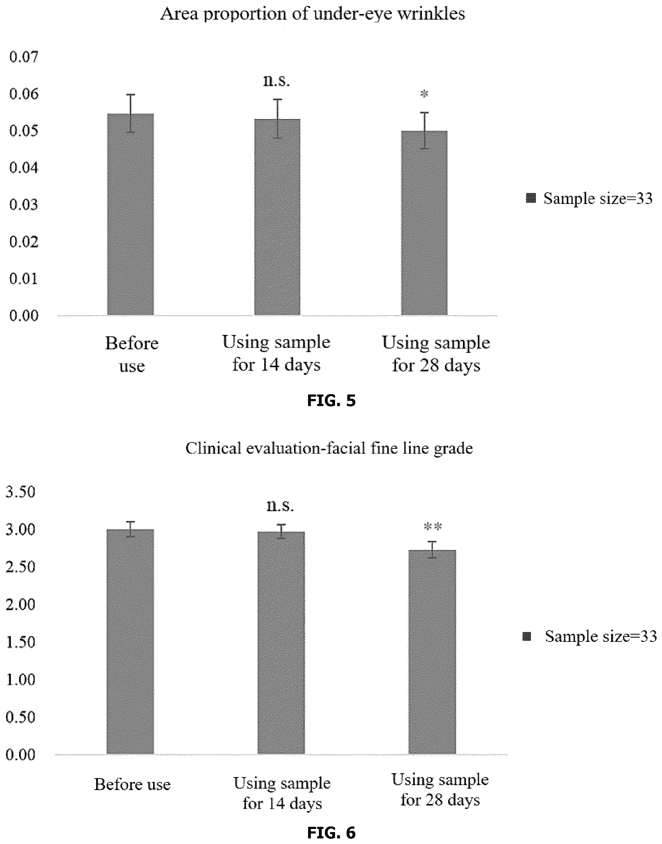

FIG. 5 is a comparison chart of area proportions of under-eye wrinkle before and after the transdermal composition prepared in Example 9 is used (the smaller the under-eye measurement value, the smaller the area proportion of under-eye wrinkle): significance marking method: "ns" indicates no significant difference, P ≥ 0.05; "*" indicates significant difference, 0.01 ≤ P < 0.05; "**", 0.001 ≤ P < 0.01; "***", P < 0.001.

The results showed that compared with before use, the area proportion of under-eye wrinkles was significantly reduced after 28 days of use (P<0.05), with the improvement rate of 8.46%.

**7.8 Descriptive and statistical analysis of grade test results for items such as Clinical evaluation-facial fine line (the smaller the measured value, the smaller the number of facial fine lines)**

FIG. 6 is a comparison chart of clinical evaluation-facial fine line grades before and after the transdermal composition prepared in Example 9 is used (the smaller the measured value, the smaller the area proportion of facial fine lines);

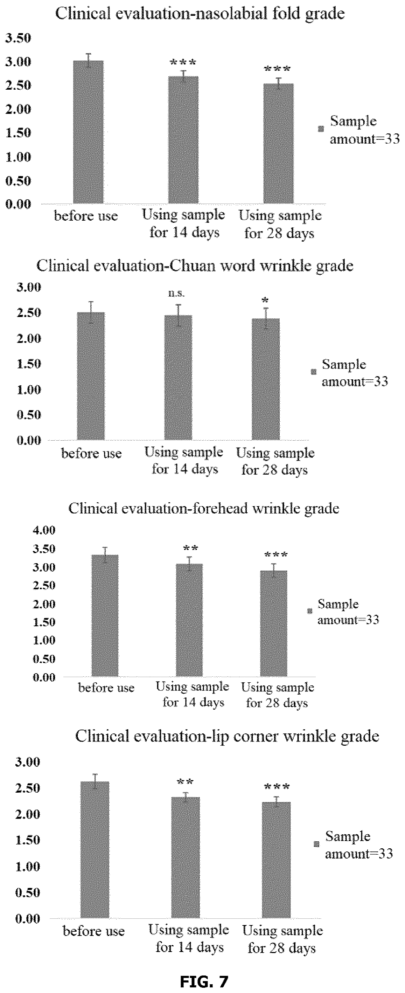

FIG. 7 is a comparison chart of clinical evaluation-nasolabial fold grade, Chuan word wrinkle grade, forehead wrinkle grade and lip corner wrinkle grade before and after the transdermal composition prepared in Example 9 is used (the smaller the measurement value, the smaller the area proportion of fine lines).

The results show that:

compared with the situation before use, after the sample was used for 28 days, the improvement rate of clinical evaluation-facial fine line grade was 9.09%;

compared with the situation before use, after the sample was used for 14 days, the improvement rate of clinical evaluation-nasolabial fold grade was 11.00%, which was a significant difference;

compared with the situation before use, after the sample was used for 28 days, the improvement rate of clinical evaluation - nasolabial fold grade was 16.00%, with a significant difference;

compared with the situation before use, after the sample was used for 28 days, the improvement rate of the clinical evaluation-Chuan word wrinkle grade was 4.82%, with a significant difference;

compared with the situation before use, after the sample was used for 14 days, the improvement rate of clinical evaluation-forehead wrinkle grade was 7.27%, with a significant difference;

compared with the situation before use, after the sample was used for 28 days, the improvement rate of clinical evaluation-forehead wrinkle grade was 12.73%, with a significant difference.

compared with the situation before use, after the sample is used for 14 days, the improvement rate of the clinical evaluation-lip corner wrinkle grade was 11.63%, with a significant difference; and

compared with the situation before use, after the sample was used for 28 days, the clinical evaluation-lip corner wrinkle grade improvement rate was 15.12%, with a significant difference.

**Test conclusion**

The test sample was applied to 33 subjects for 28 consecutive days. The following effects can be seen based on the results:

The instrument detection results show:

1) compared with the situation before use, after the sample was used for 14 days and 28 days, the moisture contents in the skin stratum corneum were significantly improved (P<0.05), with improvement rates of 21.23% and 41.38% respectively;

2) compared with the situation before use, after the sample was used for 14 days and 28 days, the transepidermal water loss rates of the skin were significantly reduced ($P<0.05$), with improvement rates of 15.84% and 28.73% respectively;

3) compared with the situation before use, after the sample was used for 14 days and 28 days, the skin elasticity R2 values were significantly improved ($P<0.05$), with improvement rates of 11.02% and 20.13% respectively;

4) compared with the situation before use, after the sample was used for 14 days and 28 days, the skin firmness F4 values were significantly reduced ($P<0.05$), with improvement rates of 8.45% and 18.35% respectively; and

5) compared with the situation before use, after the sample was used for 28 days, the areaproportion of under-eye wrinkle of the skin was significantly reduced ($P<0.05$), with an improvement rate of 8.46%.

The visual evaluation results show:

1) compared with the situation before use, after the sample was used for 28 days, the clinical evaluation-facial fine line grade was significantly reduced ($P<0.05$), with an improvement rate of 9.09%;

2) compared with the situation before use, after the sample was used for 14 days and 28 days, the clinical evaluation-nasolabial fold grades were significantly reduced ($P<0.05$), with improvement rates of 11.00% and 16.00% respectively;

3) compared with the situation before use, after the sample was used for 28 days, the clinical evaluation-Chuan word wrinkle grade was significantly reduced ($P<0.05$), with an improvement rate of 4.82%;

4) compared with the situation before use, after the sample was used for 14 days and 28 days, the clinical evaluation-forehead wrinkle grades were significantly reduced ($P<0.05$), with improvement rates of 7.27% and 12.73% respectively; and

5) compared with the situation before use, after the sample was used for 14 days and 28 days, the clinical evaluation-lip corner line grades were significantly reduced ($P<0.05$), with improvement rates of 11.63% and 15.12%, respectively.

Subject's subjective evaluation results show:

1) After the sample was used for 28 days, 96.97% of the subjects generally agreed with the sample;

2) After the sample was used for 28 days, 100% of the subjects considered that the sample was mild and non-irritating.

The safety assessment results showed that: after 33 subjects used the sample for 28 days, no adverse reactions were observed on their skins.

**Example 11**

The transdermal compositions prepared in Examples 1-6 and 9 were: transparent and clear, and:

(1) Particle size detection: the transmission electron microscopy picture showed that the particle size was 100-200 nm (as shown in FIG. 1);

(2) Stability detection: after centrifugation at 10,000 rpm for 5 min, there was no stratification, indicating excellent stability;

(3) Water-in-oil performance:

For each sample, two emulsions (transdermal compositions) having the same volume were taken, and two drops of Sudan Red III dye and methylene blue dye solution were simultaneously and respectively added into the emulsions for standing. The diffusion speed and appearance change of the two dyes (Sudan Red III-red and methylene blue-blue) in the emulsions were observed:

the Sudan Red III dye is an oil-soluble dye and easily diffuses in an oil phase; the methylene blue dye is a water-soluble dye and easily diffuses in an aqueous solution;

the standard for determining the type of the emulsion is that if the diffusion speed of blue is greater than the diffusion speed of red, it is an O/W type emulsion; on the contrary, it is a W/O type emulsion.

The test results showed that the prepared transdermal compositions were water-in-oil type emulsions

**Comparative examples 1-3**

In Comparative examples 1-3, other matrices were used; see Table 25 for details.

| Table 25 | | | |
|---|---|---|---|
| In percentage by mass based on the total weight | | | |
| Name of composition | Comparative example 1 | Comparative example 2 | Comparative example 3 |
| Oleic acid | 9.50% | 14.30% | 11.80% |
| Oleyl alcohol | 9.50% | 14.30% | 9.80% |
| Propylene glycol | 4.80% | 4.80% | 9.80% |
| Polysorbate 80 | 9.50% | 4.80% | 19.60% |
| Sorbitan oleate | 9.50% | 4.80% | 19.60% |
| Purified water | 57.20% | 57.00% | 29.40% |

The proportioning results showed that compared with each example, the comparative examples exhibited a poor emulsification effect.

**Claims**

1.  A transdermal composition:

    comprising an active substance capable of exerting a predetermined effect,
    wherein the transdermal composition is in the form of an emulsion, and based on the total weight of the transdermal composition in percentage by mass, in the transdermal composition:

    the percentage of oil phase is: 15%-50%, preferably 30%-45% or 35%-40%;
    the percentage of emulsifier is: 35%-65%, preferably 40%-50%;
    the percentage of aqueous phase is: 10%-35%, preferably 10%-25%.

2.  The transdermal composition according to claim 1, wherein:
    wherein, the emulsion has a particle size distribution of 0.01 $\mu$m-1 $\mu$m, or 100-200 nm.

3.  The transdermal composition according to claim 1 or 2, wherein:
    wherein, by mass ratio, the oil phase to the aqueous phase to the emulsifier is: 2-3:2.4-4:1 or 2-3:2.4-3.7:1.

4.  The transdermal composition according to any one of claims 1 to 3, wherein:
    based on the total weight of the transdermal composition in percentage by mass, a matrix component of the emulsion comprises: 1%-15% of oleic acid; 10%-30% of emulsifying stabilizer, 5%-15% of any one or more of propylene glycol and butylene glycol, 0.5%-4% of menthol, 1%-5% of laurocapram, 35-65% of emulsifier, and 10-20% of water.

5.  The transdermal composition according to claim 4, wherein:

    wherein, the emulsifier comprises sorbitan oleate and any one or more selected from polysorbate 80, polysorbate 60 and polysorbate 20,
    and in percentage by mass:
    the sorbitan oleate accounts for 10%-15% of the total weight of the transdermal composition, and any one or more of polysorbate 80, polysorbate 60 and polysorbate 20 accounts for the total weight of the transdermal composition is 25%-50% or 30%-40%.

6.  The transdermal composition according to claim 4 or 5, wherein:
    wherein, based on the total weight of the transdermal composition in percentage by mass, the matrix component of the emulsion comprises: 1%-15% of oleic acid; 10%-30% of oleyl alcohol, 5%-15% of propylene glycol or butylene glycol, 0.5%-4% or 1%-2% of menthol, 1%-5% or 2%-4% or 2% of laurocapram, 10%-15% of sorbitan, and 30%-40% of any one or more of polysorbate 80, polysorbate 60 and polysorbate 20, and 10%-20% of water.

7. The transdermal composition according to any one of claims 2 to 5:

comprising a preservative,
preferably, based on the total weight of the transdermal composition in percentage by mass, the transdermal composition comprises 0.1%-1% of preservative;
furthermore, the preservative is selected from any one or more of phenoxyethanol, methylparaben, ethylparaben, propylparaben, benzyl alcohol and sorbic acid.

8. The transdermal composition according to any one of claims 1 to 6, wherein:

wherein, the active substance contains a biomolecular substance,
preferably, the biomolecule active substance is less than or equal to approximately 300 kDa or less than or equal to approximately 150 kDa.

9. The transdermal composition according to claim 8, wherein:

wherein, the biomolecular substance has a structure capable of achieving the following functions: an exocytosis fusion machinery capable of being transported to a target cell and cleave the target cell,
furthermore, the biomolecular substance is a clostridial neurotoxin; preferably, the mass percentage of the clostridial neurotoxin based on the total weight of the transdermal composition is less or equal to 0.02%; more preferably less or equal to 0.015%; even more preferably less or equal to 0.012%; even furthermore, the clostridial neurotoxin is a type A, B, C, D, E, F or G botulinum toxin, and/or a light chain of the clostridial neurotoxin comprises an amino acid sequence having at least 35%, 50%, 60%, 70%, 75%, 80%, 85%, 90%, 99% or 100% identity to any one of SEQ ID NOs: 1-7; and/or a heavy chain of the clostridial neurotoxin comprises an amino acid sequence having at least 35%, 50%, 60%, 70%, 75%, 80%, 85%, 90%, 99% or 100% identity to any one of SEQ ID NOs: 8-14.

10. The transdermal composition according to claim 8 or 9, wherein:
wherein, the composition of the transdermal composition is shown in Table 6.

11. The transdermal composition according to any one of claims 1 to 9, wherein:
wherein, the active substance comprises any one or more of the following: a yeast extract, sodium ascorbyl phosphate, *Hippophae rhamnoides* seed oil, optional vitamins, retinyl palmitate, an aloe extract, allantoin, *Glycyrrhiza glabra* extract, a ginseng extract, a *Rheum palmatum* extract, a *Centella asiatica* extract, a tea extract and a *Hamamelis virginiana* extract; preferably, the composition of the transdermal composition is shown in Table 7.

12. A use of the transdermal composition according to any one of claims 1 to 11 in achieving therapeutic, cosmetic and skin care effects.

13. The use according to claim 12, wherein: when being used, transdermal use is performed in any one or more of applying, a transdermal instrument, patching and spraying.

14. A use of the transdermal composition according to any one of claims 1 to 11 in the preparation of a medicament or product for achieving therapeutic, cosmetic and skin care effects.

**FIG. 1**

Left leg of a mouse treated without botulinum toxin protein A microemulsion

Right leg of a mouse treated with botulinum toxin protein A microemulsion

**FIG. 2**

Skin elasticity R2 value

**FIG. 3**

Skin firmness F4 value

**FIG. 4**

Area proportion of under-eye wrinkles

**FIG. 5**

Clinical evaluation-facial fine line grade

**FIG. 6**

Clinical evaluation-nasolabial fold grade

Clinical evaluation-Chuan word wrinkle grade

Clinical evaluation-forehead wrinkle grade

Clinical evaluation-lip corner wrinkle grade

FIG. 7

| Best-fit values | |
|---|---|
| Bottom | 0.8005 |
| Top | 0.0544 |
| IC50 | 257.9 |
| HillSlope | 1.287 |
| logIC50 | 2.411 |
| Span | -0.7461 |

FIG. 8

## INTERNATIONAL SEARCH REPORT

| International application No. |
| --- |
| **PCT/CN2024/078986** |

| A. | CLASSIFICATION OF SUBJECT MATTER |
| --- | --- |
| | A61K9/107(2006.01)i;  A61K47/00(2006.01)i;  A61K8/64(2006.01)i;  A61K38/48(2006.01)i |

According to International Patent Classification (IPC) or to both national classification and IPC

| B. | FIELDS SEARCHED |
| --- | --- |

Minimum documentation searched (classification system followed by classification symbols)

    IPC: A61K

Documentation searched other than minimum documentation to the extent that such documents are included in the fields searched

Electronic data base consulted during the international search (name of data base and, where practicable, search terms used)

    CNABS, CNTXT, DWPI, WPABS, ENTXT, CJFD, STNext, ISI Web of Science, Pubmed, 百度学术, Baidu Scholar, 中国期刊全文数据库, Chinese Journal Full-text Database: 康码(上海)生物科技有限公司, 郭敏, 江铸颜, 刘章, 肖嵩, 冯燕, 于雪, 透皮, 乳剂, 微乳, 纳米乳, 油相, 水相, 乳化剂, 表面活性剂, 油酸, 油醇, 丙二醇, 丁二醇, 羧菌, 肉毒菌素, 山梨坦油酸酯, 司盘, 聚山梨醇, 吐温, 薄荷醇, 月桂氮卓酮, oleic acid, oleyl alcohol, Tween, Span, Azone, butanediol, propylene glycol, tranzone, emulsion, emulsifier, surfactant, Botox, botulin, botulinum

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 114452255 A (CHANGZHOU HENGBANG PHARMACEUTICAL CO., LTD.) 10 May 2022 (2022-05-10) description, paragraphs [0003], [0034] and [0102] | 1-7, 11-14 |
| Y | CN 114452255 A (CHANGZHOU HENGBANG PHARMACEUTICAL CO., LTD.) 10 May 2022 (2022-05-10) description, paragraphs [0003], [0034] and [0102] | 8-10, 12-14 |
| Y | US 2014099342 A1 (UNIVERSITY OF MASSACHUSETTS LOWELL) 10 April 2014 (2014-04-10) claims 181 and 187, and description, paragraphs [0002] and [0069] | 8-10, 12-14 |
| X | US 2005232953 A1 (BARNIKOL, W. et al.) 20 October 2005 (2005-10-20) claims 1-23, and description, table 2, formulation 5, and paragraphs [0001] and [0104] | 1-14 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **20 May 2024** | **24 May 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/078986** |

**C.    DOCUMENTS CONSIDERED TO BE RELEVANT**

| Category* | Citation of document, with indication, where appropriate, of the relevant passages | Relevant to claim No. |
|---|---|---|
| X | CN 113960208 A (JINAN LIANGFU JINGHE PHARMACEUTICAL TECHNOLOGY CO., LTD.) 21 January 2022 (2022-01-21)<br>    description, paragraphs [0002]-[0003], [0019], [0031] and [0040] | 1-7, 11-14 |
| X | CN 104856954 A (SICHUAN JIUZHANG BIOLOGICAL SCIENCE AND TECHNOLOGY CO., LTD.) 26 August 2015 (2015-08-26)<br>    claims 1-10 | 1-7, 11-14 |
| X | US 2002155084 A1 (THE REGENTS OF THE UNIVERSITY OF THE MICHIGAN) 24 October 2002 (2002-10-24)<br>    claims 14-16, embodiments 5-6, table 3 (1:1 lean oil), and figure 11 | 1-7, 11-14 |
| A | CHEN, H. et al. "A Study of Microemulsion Systems for Transdermal Delivery of Triptolide"<br>*Journal of Controlled Release,* Vol. 98, No. (3), 14 June 2004 (2004-06-14),<br>    pp. 426-436 | 1-14 |

Form PCT/ISA/210 (second sheet) (July 2022)

**INTERNATIONAL SEARCH REPORT**

| International application No. |
| --- |
| **PCT/CN2024/078986** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/078986** |

**Box No. II        Observations where certain claims were found unsearchable (Continuation of item 2 of first sheet)**

This international search report has not been established in respect of certain claims under Article 17(2)(a) for the following reasons:

1. ☑ Claims Nos.: **12**
   because they relate to subject matter not required to be searched by this Authority, namely:

   The technical solution of claim 12 comprises the use of a transdermal composition in achieving treatment, which relates to a method for treatment of a human body; therefore, said claim does not comply with PCT Rule 39.1(iv). The search report is provided on the basis that the designation of the subject matter thereof is a corresponding pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/078986**

| Patent document cited in search report | | | Publication date (day/month/year) | Patent family member(s) | | | Publication date (day/month/year) |
|---|---|---|---|---|---|---|---|
| CN | 114452255 | A | 10 May 2022 | None | | | |
| US | 2014099342 | A1 | 10 April 2014 | US | 2017181952 | A1 | 29 June 2017 |
| | | | | US | 10576034 | B2 | 03 March 2020 |
| | | | | US | 9486408 | B2 | 08 November 2016 |
| | | | | US | 2017312210 | A1 | 02 November 2017 |
| | | | | US | 10532019 | B2 | 14 January 2020 |
| | | | | US | 2020214961 | A1 | 09 July 2020 |
| | | | | US | 2022054389 | A1 | 24 February 2022 |
| US | 2005232953 | A1 | 20 October 2005 | ATE | 352276 | T1 | 15 February 2007 |
| | | | | DE | 10226990 | A1 | 18 March 2004 |
| | | | | WO | 03105797 | A1 | 24 December 2003 |
| | | | | DE | 50306384 | D1 | 15 March 2007 |
| | | | | ES | 2281651 | T3 | 01 October 2007 |
| | | | | EP | 1513492 | A1 | 16 March 2005 |
| | | | | EP | 1513492 | B1 | 24 January 2007 |
| | | | | AU | 2003276914 | A1 | 31 December 2003 |
| CN | 113960208 | A | 21 January 2022 | None | | | |
| CN | 104856954 | A | 26 August 2015 | None | | | |
| US | 2002155084 | A1 | 24 October 2002 | WO | 0191728 | A2 | 06 December 2001 |
| | | | | WO | 0191728 | A3 | 25 April 2002 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GERALD K**. Cell and Molecular Biology. John Wiley & Sons, Inc, 2002
- **TEMERI D WILDER-KOFIE et al.** *An Alternative In Vivo Method to Refine the Mouse Bioassay for Botulinum Toxin Detection*, June 2011, vol. 61 (3)

- Cosmetic safety technical specifications. 2015